# EUROPEAN PATENT APPLICATION

(11) **EP 3 943 555 A1**
(43) Date of publication of application: **26.01.2022**
(21) Application number: 20773239.7
(22) Date of filing: 16.03.2020
(51) Int. Cl.: C08L 101/02, C08L 71/02, C12M 1/00, C12M 3/00, C12M 3/04, C12N 5/07, C12N 5/071, C08G 75/04, C12N 11/04

(54) **CELL CULTURE CARRIER, AND METHOD AND DEVICE FOR PRODUCING SAME**

(30) Priority: 20.03.2019 JP 2019053871; 09.10.2019 JP 2019186411; 29.11.2019 JP 2019217131
(71) Applicant: Ricoh Company, Ltd., Tokyo 143-8555 (JP)
(72) Inventor: SATOH Naoki, Tokyo 143-8555 (JP); YAGINUMA Hidekazu, Tokyo 143-8555 (JP); ARATANI Tomoyuki, Tokyo 143-8555 (JP); SHIONOIRI Momoko, Tokyo 143-8555 (JP); IWASHITA Natsuko, Tokyo 143-8555 (JP); SAMESHIMA Tatsuya, Tokyo 143-8555 (JP); YAMAZAKI Takehiro, Tokyo 143-8555 (JP)
(74) Representative: J A Kemp LLP
(86) International application number: PCT/JP2020/011554
(87) International publication number: WO 2020/189645

(57) **Abstract**

This invention is to develop a production method for a cell culture carrier comprising a hydrogel having high shape retainability, and cell culture carrier production device, and to provide a structure composed of a cell culture carrier produced using the same.

The production method comprises a retention step of retaining on a support material a first solution containing a multiple branching polymer comprising polyethylene glycol as the backbone, and having at least one functional group which is one of a nucleophilic functional group and an electrophilic functional group at a side chain(s) and/or a terminal(s), and a gel formation step of forming one or more dot-shaped hydrogels in which a second solution containing a multiple branching polymer comprising polyethylene glycol as the backbone, and having at least one functional group which is the other one of a nucleophilic functional group and an electrophilic functional group at a side chain and/or a terminal, is discharged by a droplet discharge device to land in contact with the first solution retained on the support material.

## Description

### Technical Field

The present invention relates to a cell culture carrier, a production method, and production device for the same.

### Background Art

In step with the progress of the stem cell technology and tissue engineering in recent years, development of a three-dimensional structure artificially formed a tissue consisting of a plurality of cells has been progressed. As the cell formation technology for forming a three-dimensional structure, a cell sheet method (Patent Literature 1), a spheroid stacking method (Patent Literature 2), a gel extrusion method (Patent Literature 3), an inkjet method (Patent Literature 4), etc. are known. Among others, a bioprinter method, such as a gel extrusion method and an inkjet method, has been attracting attention in construction of a three-dimensional structure, because cells can be quickly fixed on the bottom of a culture vessel, or on a gel.

The "gel extrusion method" is a method for producing a three-dimensional structure by continuously extruding a gel containing cells from a nozzle and stacking the cells. Although it is easy to stack gels, there is a problem that high-resolution cell placement is not possible, because the cell placement unit is several hundred micrometers or more. Since the resolution of the cell placement depends on the nozzle diameter, it is possible to improve the resolution by reducing the nozzle diameter, however there arises a new problem that the shear damage worked on the cells increases.

Meanwhile, the "inkjet method" is capable of constructing a three-dimensional structure with high resolution by discharging an ink containing cells from an inkjet head to be placed with high accuracy. However, the ink to be used needs to have a low viscosity in order to avoid clogging of the inkjet head. For example, Patent Literature 4 discloses a method for producing a gel three-dimensional structure, by which an aqueous solution of sodium alginate containing cells is discharged as an inkjet into an aqueous solution of calcium chloride to cause gelation. Since an aqueous solution of sodium alginate has a low viscosity, and can be designed to shorten the gelation time, three-dimensional placement of cells is possible and easy. However, since the gel three-dimensional structure disclosed in Patent Literature 4 is produced in a liquid, and the gel is not fixed to a support material, such as a slide glass, or a culture vessel, there has been drawbacks in that the application of the produced gel three-dimensional structure is restricted, and the reproducibility of the cell placement is low. In contrast, for example, Patent Literature 5 discloses a method for producing a gel having a three-dimensional structure with three-dimensional placement. By this method, an aqueous solution of sodium alginate containing cells is discharged to an aqueous solution of calcium chloride on a slide glass. The aqueous solution of sodium alginate is composed of low molecular weight materials, and by selecting raw materials, the viscosity can be made low and the gelation time can be made short, so that highly accurate three-dimensional placement becomes possible. However, since gelation is achieved by ionic crosslinking between alginic acid and a chloride, during immersion in a buffer solution or a culture medium for cells, the chlorides in the gel may be gradually removed leading to decomposition of the gel, and therefore its shape can be hardly retained. Further, it is known that cells encapsulated in an alginate gel cannot survive for a long period of time in the gel. Although it is conceivable to decompose the gel with EDTA or the like, the gel may be instantaneously decomposed, which may break the cell placement, and there arises concern about its impact on cells.

Patent Literature 6 discloses a method for producing a gel three-dimensional structure by discharge a liquid containing gelatin or fibrinogen. A liquid adjusted to a low concentration of gelatin or fibrinogen can be discharged from an inkjet head. However, the gelation time of gelatin or fibrinogen adjusted to a low concentration is so long that cells sediment under their own weight even if the cells are placed three-dimensionally, and therefore, it is difficult to place cells arbitrarily.

### Citation List

### Patent Literature

Patent Literature 1: Japanese Patent No. 5322333
Patent Literature 2: Japanese Unexamined Patent Application Publication No. 2017-101015
Patent Literature 3: Japanese Translation of PCT International Application Publication No. 2014-531204
Patent Literature 4: Japanese Patent No. 4974144
Patent Literature 5: Japanese Unexamined Patent Application Publication No. 2019-17255
Patent Literature 6: Japanese Unexamined Patent Application Publication No. 2017-209103

### Brief Description of Drawings

[Figure 1] Figure 1 is a flow chart of the production method for a cell culture carrier of the present invention.
[Figure 2] Figure 2 is a schematic diagram of a solution discharge head which is a discharge means of the present invention.
[Figure 3] Figure 3 is examples of the input waveform to the second solution discharge head.
[Figure 4] Figure 4 is a schematic diagram of the production device for a cell culture carrier of the present invention provided with a plurality of solution discharge heads.
[Figure 5] Figure 5 is schematic diagrams of cell culture carriers formed in various shapes on the support material by the production device for a cell culture carrier of the present invention.
[Figure 6] Figure 6 is a diagram showing an example of the production method for a cell culture carrier of the present invention.
[Figure 7] Figure 7 shows examples of a hydrogel which is formed by controlling contiguity of dot-shaped hydrogels with a solution discharge head which discharge velocity is regulated. (a) shows a patterning of dot-shaped hydrogels connected linearly by decreasing little by little the pitches between dot-shaped hydrogels. (b) shows a patterning of curved linear hydrogel formed by connecting dots on a concentric circumference.
[Figure 8] Figure 8 is an example of a removal means of the present invention.

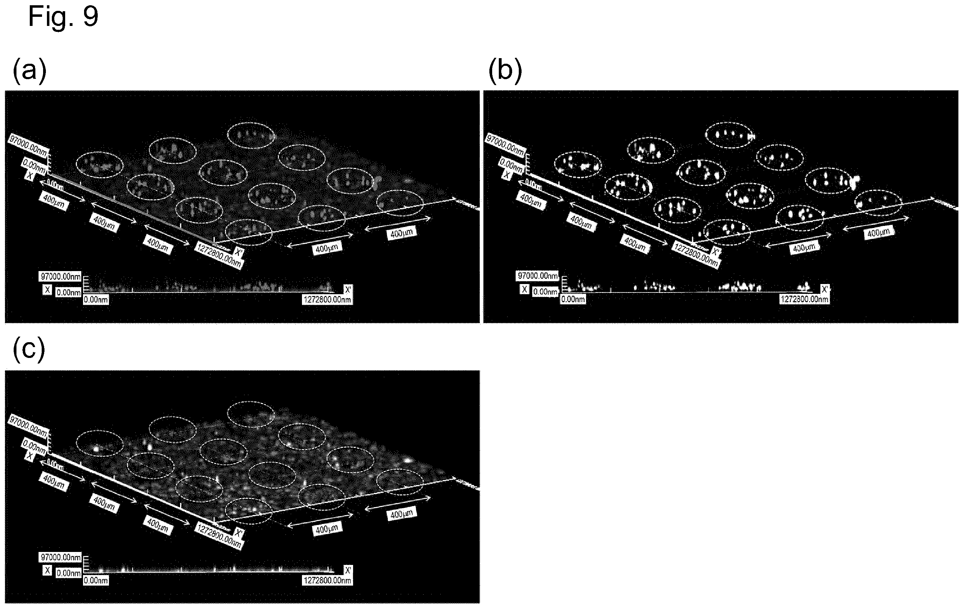

[Figure 9] Figure 9 is an example of stacked hydrogels. (a) is a perspective view of hydrogels illustrating all of the 2 stacked layers, (b) is a perspective view of hydrogels illustrating only the second layer of the 2 layers, and Figure 9(c) is a perspective view of hydrogels illustrating only the first layer of the 2 layers. Each point in the figure represents a stained cell, and the broken line circle indicates the range in which droplets of a cell-containing second solution are dropped one by one at a pitch of 400 µm. Therefore, in (a), each point outside the broken line circle represents a cell in the first layer, and each point inside the broken line circle represents a cell in the second layer.
[Figure 10] Figure 10 is an example of stacked hydrogels. (a) is a cross-sectional view of hydrogels illustrating all of the 4 stacked layers, (b) is a cross-sectional view of hydrogels illustrating only the second and fourth layers of the 4 layers, and (c) is a cross-sectional view of hydrogels illustrating only the first and third layers of the 4 layers.
[Figure 11] Figure 11 is an example of stacked hydrogels. (a) is a cross-sectional view of hydrogels illustrating all of the 10 stacked layers, (b) is a cross-sectional view of hydrogels illustrating the odd-numbered layers from the first to ninth layer of 10 layers, and (c) is a cross-sectional view of hydrogels illustrating the even-numbered layers from the second to tenth layer of 10 layers.
[Figure 12] Figure 12 is an example of stacked hydrogels. (a) is a cross-sectional view of hydrogels illustrating all of the 3 stacked layers, (b) is a cross-sectional view of hydrogels illustrating all of the 3 layers containing the first kind of cells, and (c) is a cross-sectional view of hydrogels illustrating only the second layer of 3 layers containing the second kind of cells.
[Figure 13] Figure 13 is an example of stacked hydrogels. (a) is a cross-sectional view of hydrogels illustrating all of the 20 stacked layers, (b) is a cross-sectional view of hydrogels illustrating the odd-numbered layers from the first to nineteenth layer of 20 layers, and (c) is a cross-sectional view of hydrogels illustrating the even-numbered layers from the second to twentieth layer of 20 layers.
[Figure 14] Figure 14 is a schematic diagram of hydrogels with a two-layer structure in which a tetra-PEG gel is formed in the first and second layers and stacked. (a) is a top view, and (b) is a side view of one of the dots shown in (a).
[Figure 15] Figure 15 is a schematic diagram of hydrogels with a two-layer structure in which a tetra-PEG gel is formed as the first layer and an alginate gel or a fibrin gel is stacked as the second layer. (a) is a top view, and (b) is a side view of one of the dots shown in (a).

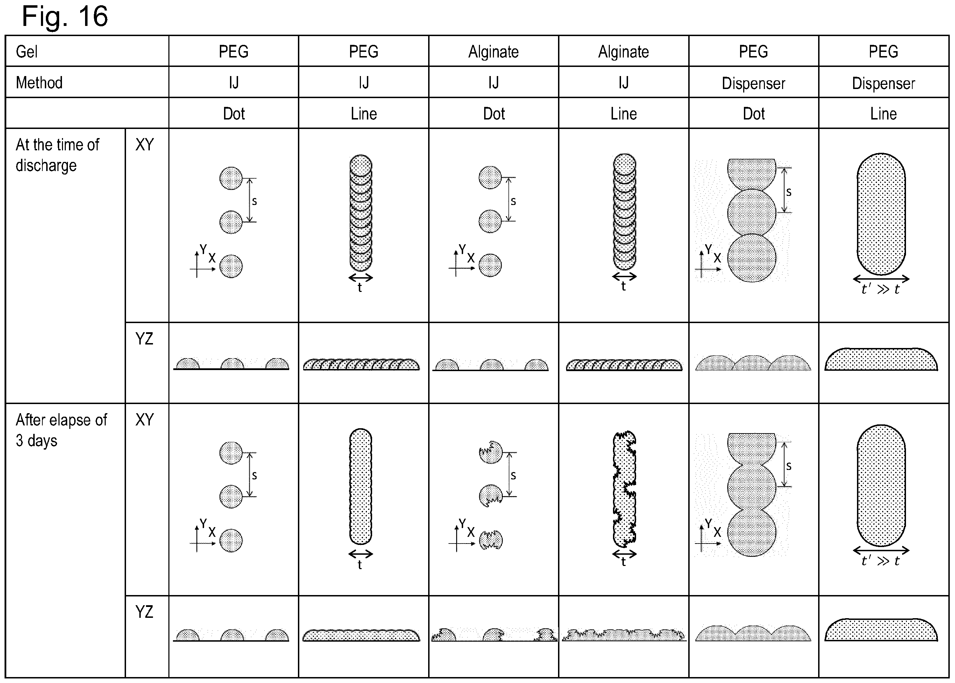

[Figure 16] Figure 16 is a chart concerning the retained shapes of gels, when hydrogels are stacked under various conditions. The gel component (PEG, and alginate), discharge method (inkjet method, and dispenser method), gel shape (dot-shaped, and linear), and elapse of time after gel formation (at the time of discharge, and after an elapse of 3 days) are respectively indicated. XY means a top view, and X and Y indicate the horizontal axis and the vertical axis, respectively. YZ means a side view, and Z indicates the depth.
[Figure 17] Figure 17 is a cross-sectional view of a cell culture carrier prepared by adding fibrinogen and thrombin as cell acting additives into the first solution and the second solution respectively. PBS was used as the dispersion medium of the first solution and the second solution. In the figure, the white part is fibrin present in a hydrogel. In addition, the "top" indicates the upper part of the cell culture carrier, and the "bottom" indicates the lower part.
[Figure 18] Figure 18 is a cross-sectional view of a cell culture carrier prepared by adding thrombin and fibrinogen as cell acting additives into the first solution and the second solution respectively. DEME was used as the dispersion medium of the first solution. In the figure, the white part is fibrin present in a hydrogel. In addition, the "top" indicates the upper part of the cell culture carrier, and the "bottom" indicates the lower part.
[Figure 19] Figure 19 is a graph illustrating the time dependence (time-dependent change) of the permeability in a hydrogel.

### Summary of Invention

### Technical Problem

Considering the aforementioned problems, the present invention intends to to develop a production method for a cell culture carrier comprising a hydrogel with high shape retainability, and production device for a cell culture carrier, and to provide a structure comprising a cell culture carrier produced using the same.

### Solution to Problem

As a result of intensive research in order to achieve the above object, the present inventors have come to provide the following inventions.

(1) A production method for a cell culture carrier comprising a retention step of retaining a first solution containing a multiple branching polymer with one or more nucleophilic functional groups or electrophilic functional groups at a side chain(s) and/or a terminal(s), comprising polyethylene glycol as the backbone, and a gel formation step of forming one or more hydrogels by landing a droplet of a second solution discharged from a droplet discharge device in the first solution so that the droplet contacts with the retained first solution, wherein the second solution contains a multiple branching polymer with one or more other nucleophilic functional groups or electrophilic functional groups at a side chain(s) and/or a terminal(s), comprising polyethylene glycol as the backbone.

This application claims the priority based on Japanese Patent Applications No. 2019-053871, 2019-186411, and 2019-217131, the disclosure contents of which are incorporated herein in its entirety.

### Advantageous Effects of Invention

According to the present invention, it is possible to provide a cell culture carrier comprising a hydrogel with high shape-retainability, as well as a production method and production device for the same,

### Description of Embodiments

### 1. Production method for cell culture carrier

### 1-1. Outline

A first aspect of the present invention is a production method for a cell culture carrier. The production method of the present invention is a method for forming a thick cell culture carrier, using a solution containing a multiple branching polymer comprising as the backbone either of polyethylene glycol with one or more nucleophilic functional groups at a side chain(s) and/or a terminal(s), or polyethylene glycol with one or more electrophilic functional groups at a side chain(s) and/or a terminal(s), and a solution containing a multiple branching polymer comprising as the backbone polyethylene glycol with the other functional groups, which gelates with the prior multiple branching polymer by a gelation reaction, by discharging droplets of a solution containing one of the polymers with a droplet discharge device to land in contact with a solution containing the other polymer so as to gelate. Since it is possible to place hydrogels three-dimensionally with high resolution and high accuracy by the production method of the present invention, a cell culture carrier ensuring high reproducibility and excellent shaping property can be produced using hydrogels as a cell support material.

### 1-2. Definition of terms

The following terms frequently used herein will be defined.

(1) Multiple branching polymer comprising polyethylene glycol as the backbone Herein a "multiple branching polymer comprising polyethylene glycol as the backbone" (herein often simply referred to as "multiple branching polymer" or "multi-arm PEG") is a polymer used as a gelling material. When two types of multi-arm PEGs with respectively a nucleophilic functional group(s) and an electrophilic functional group(s) at the terminals of a plurality of polyethylene glycol (PEG) branches are crosslinked together, a gel with a network structure (multi-arm PEG gel) can be formed. For example, in a case of two types of 4-fold branched polymers (herein often referred to as "tetra-PEG") with respectively a nucleophilic functional group and an electrophilic functional group at the terminals of 4 PEG branches, a gel called "tetra-PEG gel" having a uniform network structure can be formed. There is no particular restriction on the number of the branches of a multiple branching polymer. Normally, it is acceptable, if PEG has three or more branches with an electrophilic terminal and a nucleophilic terminal, and the number may be appropriately selected as needed. Two or more PEGs constituting a multi-arm PEG may have different numbers of branches, as long as they have respectively a nucleophilic functional group and an electrophilic functional group.

It has been reported that among multi-arm PEGs, a tetra-PEG gel has an ideally uniform network structure (Matsunaga et al., Macromolecules, Vol. 42, No. 4, pp.1344-1351, 2009), but not limited thereto. In addition, a tetra-PEG gel can be formed on the spot easily and rapidly by simply mixing two types of tetra-PEGs (included in the first solution and the second solution of the present invention). Furthermore, the gelation time can be controlled by adjusting the pH or concentration of each tetra-PEG. In addition, since it contains PEG as the main component, it has excellent biocompatibility. When a solution containing cells and a tetra-PEG is discharged by a droplet discharge device so that two types of tetra-PEGs are reacted to mold a tetra-PEG gel, the cells can be placed three-dimensionally.

An embodiment of the tetra-PEG of the present invention is a compound with a structure represented by the following Formula (I).

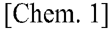

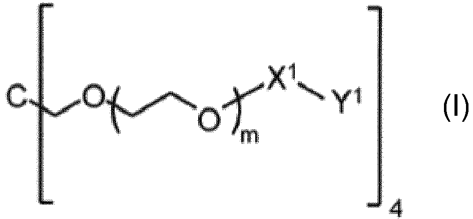

The "m" in Formula (I) may be the same or different. The more analogous the values of m are, the more uniform the three-dimensional structure can become, and the higher the strength of the gel becomes. Therefore, in order to yield a high-strength gel, the same m values are preferable. When the m value is too high the strength of the gel becomes weak, and when each m value is too low, it becomes difficult to form a gel due to steric hindrance of the compound. Therefore, each m is, for example, an integer of from 25 to 250, preferably from 35 to 180, more preferably from 50 to 115, and particularly preferably from 50 to 60. The molecular weight of the tetra-PEG is, for example, from 5 × 10³ to 5 × 10⁴ Da, preferably from 7.5 × 10³ to 3 × 10⁴ Da, and more preferably from 1 × 10⁴ to 2 × 10⁴ Da.

In Formula (I), "X¹" is a linker moiety that connects a functional group and the core portion. Each X¹ may be the same or different, but is preferably the same in order to produce a high-strength gel having a uniform three-dimensional structure. X¹ represents a C₁-C₇ alkylene group, a C₂-C₇ alkenylene group, -NH-Ra-, -CO-Ra-, -Rb-O-Rc-, -Rb-NH-Rc-, -Rb-CO₂-Rc-, -Rb-CO₂-NH-Rc-, -Rb-CO-Rc-, or -Rb-CO-NH-Rc-. Here, Ra represents a C₁-C₇ alkylene group, Rb represents a C₁-C₃ alkylene group, and Rc represents a C₁-C₅ alkylene group.

The "C₁-C₇ alkylene group" is an optionally branched alkylene group with 1 or more and 7 or less carbon atoms, and includes a linear chain C₁-C₇ alkylene group, and a C₂-C₇ alkylene group with one or more branches (the carbon atoms with the branches is 2 or more and 7 or less). Examples of the C₁-C₇ alkylene group comprise a methylene group, an ethylene group, a propylene group, and a butylene group. Examples of the C₁-C₇ alkylene group comprise -CH₂-, -(CH₂)₂-, -(CH₂)₃-, -CH(CH₃)-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

The "C₂-C₇ alkenylene group" is a linear or branched chain alkenylene group with 2 to 7 carbon atoms and one or more double bonds in the chain. Examples of the same include a divalent group with double bond(s) formed by removing 2 to 5 hydrogen atoms of adjacent carbon atoms of an alkylene group.

In Formula (I), "Y¹" is a functional group for forming a network structure by a cross-end coupling reaction which is a crosslinking reaction by a covalent bond as described above, and is selected from a nucleophilic functional group or an electrophilic functional group.

As a "nucleophilic functional group", for example, but not limited to, a thiol group capable of shortening the gelation time is preferable. The functional groups may be the same or different, but it is preferable that they are the same. When the functional groups are the same, the reactivity with the nucleophilic functional groups involved in the crosslinking reaction becomes uniform, and a high-strength gel having a uniform three-dimensional structure may be obtained easier. A tetra-PEG with a nucleophilic functional group is herein expressed as "nucleophilic tetra-PEG".

As an "electrophilic functional group", for example, but not limited to, a maleimidyl group capable of shortening the gelation time is preferable. The functional groups may be the same or different, but it is preferable that they are the same. When the functional groups are the same, the reactivity with the nucleophilic functional groups involved in the crosslinking reaction becomes uniform, and a high-strength gel having a uniform three-dimensional structure may be obtained easier. A tetra-PEG with an electrophilic functional group is herein expressed as "electrophilic tetra-PEG".

A nucleophilic tetra-PEG and an electrophilic tetra-PEG may be so mixed that the molar ratio of the nucleophilic functional group to the electrophilic functional group falls within 0.5/1 to 1.5/1. Since the functional groups can react each at 1/1 to form a crosslink, it is preferable that the mixing molar ratio is as close to 1/1 as possible, and for forming a high-strength hydrogel, a range of from 0.8/1 to 1.2/1 is preferably. Meanwhile, when the pH of a dispersion medium in the first or second solution is from 5 to 10, the concentration of a tetra-PEG contained in the relevant solutions may be in a range of from 0.3 to 20%, and when the pH is from 6 to 10 it is preferably in a range of from 1.7 to 20%.

According to the present invention, the first solution may be composed of either of a nucleophilic tetra-PEG or an electrophilic tetra-PEG, and the second solution may be composed of the other tetra-PEG.

### (2) First solution

The "first solution" is an aqueous solution containing, as components, a dispersion medium and a multiple branching polymer comprising as the backbone either of polyethylene glycol with one or more nucleophilic functional groups at a side chain(s) and/or a terminal(s), or polyethylene glycol with one or more electrophilic functional groups at a side chain(s) and/or a terminal(s). It may further contain cells or a cell acting additive as necessary.

According to the production method of the present invention, there may be plural kinds of first solutions. In that case, all or part of a multiple branching polymer comprising as the backbone either of polyethylene glycol with one or more nucleophilic functional group at a side chain(s) and/or a terminal(s), or polyethylene glycol with one or more electrophilic functional group, a dispersion medium, cells and a cell acting additive contained in each first solution may be different. For example, a dispersion medium contained in the first solution a, and a dispersion medium contained in the first solution b may be the same or different. In a case in which there are plural kinds of first solutions, for example, when hydrogels are formed into a stack, the dispersion medium in each layer may be arbitrarily changed.

### (3) Second solution

The "second solution" is an aqueous solution containing, as essential components, a dispersion medium and a multiple branching polymer comprising as the backbone polyethylene glycol with one or more functional groups different from that in the first solution (nucleophilic functional group or electrophilic functional group) at a side chain(s) and/or a terminal(s). It may further contain cells or a cell acting additive as necessary. The second solution containing cells as described in Examples is herein often referred to as "cell-containing second solution".

According to the production method of the present invention, there may be plural kinds of second solutions. In that case, all or part of a multiple branching polymer with a functional group different from that in the first solution comprising polyethylene glycol with one or more nucleophilic functional groups, or electrophilic functional group at a side chain(s) and/or a terminal(s) as the backbone, a dispersion medium, cells and a cell acting additive contained in each second solution may be different. For example, cells contained in the second solution a, and cells contained in the second solution b may be the same or different. In a case in which there are plural kinds of second solutions, for example, the cell species in the second solution to be discharged to land in the gel formation step (S0102) or the gel stack formation step (S0108) described later may be arbitrarily changed.

### (4) Hydrogel

A "hydrogel" is a gel that has a three-dimensional network structure and retains a large volume of water inside the space of the network structure, and is also called water-retaining gel. The good example includes a polysaccharide gel such as agar, a protein gel such as jelly, and a super absorbent polymer gel such as an acrylic acid polymer. In general, a hydrogel can incorporate various substances into the gel through the retained water. The hydrogel herein is formed by the crosslinking reaction between the multiple branching polymers comprising polyethylene glycol, as the backbone, contained in the first and/or second solutions, respectively. In this regard, some of the hydrogels of this description contain cells and some do not. Herein, a hydrogel containing cells is often referred to as "cell-containing hydrogel", and a hydrogel not containing cells is often referred to as "cell-free hydrogel".

### (5) Cell culture carrier

A "cell culture carrier" means herein a carrier which contains hydrogels as a main component and is able to cultivate the containing cells three-dimensionally. The cell culture carrier may contain other components such as a cell acting gel in addition to hydrogels. There is no particular restriction on the shape or size of a cell culture carrier hereunder. The shape may be a three-dimensional structure, or a two-dimensional structure. In the case of a three-dimensional structure, it may be any of cubic, nearly cubic, cylindrical, nearly cubic, conical, nearly conical, spherical, spindle, unshaped, a shape simulating a tissue or an organ, or a combination of these. In the case of two-dimensional structure, it may be any of polygonal, nearly polygonal, circular, nearly circular, oval, nearly oval, unshaped, or a combination of these.

### (6) Cell acting gel

A "cell acting gel" means herein a gel containing a cell acting additive instead of a multiple branching polymer comprised in the aforementioned hydrogel.

### (7) Cells

The "cell" is herein one of the components to be comprised in the cell culture carrier of the present invention. There is no particular restriction on the kind of cells used herein, and any kind of cells may be arbitrarily selected depending on the intended purpose.

Cells can be taxonomically classified into, for example, eukaryotic cells and prokaryotic cells, or multicellular organism cells, and unicellular organism cells. In the inventions described herein, any cells can be used.

Examples of "eukaryotic cells" comprise animal cells (including vertebrate cells, and arthropod cells), plant cells, and fungal cells. In using these, the same kind of cells may be used singly, or two or more different kinds of cells may be used in combination. Animal cells are preferred, vertebrate cells are more preferred, and mammalian cells are further preferred. When cells are allowed to form a cell aggregate, cells with cell adhesiveness, which strongly adhere each other by intercellular adhesion, and are hardly isolated without a physicochemical treatment as in the case of adherent cells, are more preferable.

There is no particular restriction on the adherent cells, and they may be appropriately selected according to the intended purpose. Examples thereof comprise differentiated cells, and undifferentiated cells.

Examples of differentiated cells comprise hepatocytes which are liver parenchymal cells, stellate cell, Kupffer cells, endothelial cells, such as vascular endothelial cells, sinusoidal endothelial cells, and corneal endothelial cells, fibroblasts, osteoblasts, osteoclasts, periodontal membrane-derived cells, epidermal cells such as epidermal keratinocyte, epithelial cells, such as tracheal epithelial cells, intestinal epithelial cells, uterocervical epithelial cells, and corneal epithelial cells, mammary glandular cells, pericytes, muscle cells, such as smooth muscle cells, and cardiac muscle cells, renal cells, pancreatic islet of Langerhans cells, nerve cells, such as peripheral nerve cells, and optic nerve cells, chondrocytes, and osteocytes.

The differentiated cells may be primary cultured cells taken directly from an individual, an organ, or a tissue, or they may be subcultured cells passaged for several generations.

There is no particular restriction on the undifferentiated cells, and they may be appropriately selected according to the intended purpose. Examples thereof comprise pluripotent stem cells, such as embryonic stem cell (ES cells) and mesenchymal stem cells (MCS cells), unipotent stem cells such as unipotent vascular endothelial progenitor cells, and iPS cells.

Examples of "prokaryotic cells" comprise cells derived from eubacterium and cells derived from archaebacterium.

Cells may be comprised in one or more of the first solution, the second solution, and a cell acting gel. In any case, the environmental temperature at the time of preparation is in a range of from 4 to 40°C and particularly preferably in a range of from 15 to 37°C. Although the cells do not die immediately even when the temperature exceeds 37°C, if the temperature significantly exceeds 37°C, or if the temperature only slightly exceeds 37°C but for a long time, there is a concern that the cells may be damaged by heat. In addition, when the environmental temperature falls below 4°C, the activity of the cells tends to decrease, although the effect on life and death of the cells is smaller than that on the high temperature side.

### (8) Dispersion medium

A "dispersion medium" means a medium for dispersing or dissolving cells or a cell acting additive to be contained in the first solution, the second solution, a cell acting gel, or the like. There is no particular restriction on the medium, insofar as it is capable of dispersing cells, or dispersing or dissolving a cell acting additive. For example, a buffer solution or a cell culture medium described later are suitable, and particularly a cell culture medium is preferable. In this regard, the dispersion media contained in the first solution, the second solution, a cell acting gel, etc. may be the same or different. For example, a cell culture medium may be used as a dispersion medium for a solution or gel containing cells, or may be used for a solution or gel not containing cells.

### (9) Buffer solution

A "buffer solution" has a pH adjusted to the cells or the intended purpose, and may be appropriately selected out of known ones. Examples thereof comprise phosphate-buffered saline (hereinafter referred to as PBS(-)), HEPES buffer, and NaHCO₃/CO₂ buffer.

### (10) Cell culture medium

The "cell culture medium" (herein often referred to simply as "culture medium") contains at least components essential for sustaining cells, and forming an organism. The cell culture medium is hereunder comprised in the first or second solution, and further used for sustaining cells in a cell culture carrier formed, for forming an organism, or also for washing a cell culture carrier.

Generally, the culture media may be classified into a natural culture medium, a semisynthesized culture medium, a synthetic culture medium, etc., according to the composition, and may also be classified according to the shape or condition into a semi-solid culture medium, a liquid culture medium, a granular culture medium (hereinafter, also referred to as "powder culture medium"), etc. The type of the culture medium to be used hereunder may be any of them without particular restriction, insofar as it contains components necessary for the cells to be used.

The culture medium may be appropriately selected out of those publicly known in the art according to the cell species comprised in the solution or gel, and the application purpose of a cell culture carrier. Examples thereof comprise Dulbecco's modified Eagle's medium (DMEM), Ham F12 medium (Ham's nutrient mixture F12), D-MEM/F12 medium, McCoy's 5A medium, Eagle's MEM medium (Eagle's minimum essential medium; EMEM), αMEM medium (alpha modified Eagle's minimum essential medium; αMEM), MEM medium (minimum essential medium), RPMI1640 (Roswell Park Memorial Institute-1640) medium, Iscove's modified Dulbecco's medium (IMDM), MCDB131 medium, Williams' medium E, IPL41 medium, Fischer's medium, M199 medium, Hight Performance Medium 199, StemPro34 (produced by Thermo Fisher Scientific), X-VIVO 10 (produced by Cambrex Corporation), X-VIVO 15 (produced by Cambrex Corporation), HPGM (produced by Cambrex Corporation), StemSpan H3000 (produced by STEMCELL Technologies Inc.), StemSpanSFEM (produced by STEMCELL Technologies Inc.), Stemline II (produced by Sigma-Aldrich), QBSF-60 (produced by Quality Biological), StemProhESCSFM (produced by Thermo Fisher Scientific), Essential 8^{®} medium (produced by Thermo Fisher Scientific), mTeSR 1 or mTeSR 2 medium (produced by STEMCELL Technologies Inc.), ReproFF or ReproFF2 (produced by REPROCELL Inc.), PSGro hESC/iPSC medium (produced by System Biosciences), NutriStem^{®} medium (produced by Biological Industries), CSTI-7 medium (produced by Cell Science & Technology Institute, Inc.), MesenPRO RS medium (produced by Thermo Fisher Scientific), MF-Medium^{®} mesenchymal stem cell growth medium (produced by TOYOBO CO., LTD.), Sf-900 II (produced by Thermo Fisher Scientific), and Opti-Pro (produced by Thermo Fisher Scientific). These may be used singly or in combination of two or more kinds. Especially in the case of DMEM/F12 medium, the mixing ratio thereof is not particularly limited, however it is preferable that DMEM and F12 are mixed in terms of the weight concentration ratio of the components in a range of 6/4 to 4/6. The specific composition of each culture medium is publicly known in the art and it may be prepared based on the description in an appropriate document (e.g., Kaech S. and Banker G., 2006, Nat. Protoc., 1(5): 2406-15). Regarding, culture media marketed by manufacturers can be obtained by purchase. There is no particular restriction on the carbon dioxide concentration in a culture medium during a cell culture, and it may be appropriately selected according to the intended purpose. Usually 2% or more and 5% or less is preferable, and 3% or more and 4% or less is more preferable.

### (11) Cell acting additive

A "cell acting additive" means herein a substance that directly or indirectly acts on cells. There is no particular restriction on the action and property, and it may be appropriately selected according to the purpose. For example, an extracellular matrix protein, such as collagen, laminin, fibronectin, elastin, and fibrin, or a growth factor, such as a nerve growth factor, may be used for promoting adhesion, proliferation, and differentiation of cells. These may be used singly or in combination of two or more kinds. When a cell acting additive is used as a gel constituting component of a cell acting gel, as the cell acting additive a substance with a gelling property such as an extracellular matrix protein is preferable. For example, the aforementioned extracellular matrix proteins are suitable.

### (12) Support material

A "support material" is a member that retains the first solution. There is no particular restriction on the material of a support, insofar as it does not suppress the activity or proliferation of cells, and it may be appropriately selected according to the purpose. A material to which cells can be fixed, or a cell adhesive material can easily adhere is preferable.

The support materials can be roughly divided into organic materials and inorganic materials. Examples of the organic materials comprise a synthetic resin, a silicone-based material, a natural resin, a cellulosic structure (e.g. wood, and paper), a chitinous structure, a natural fiber (*e.g.* silk, wool, cotton, and spongin fiber). In addition, a cell layer fixed on a substrate can serve as an organic material-based support material. Examples of the synthetic resin comprise poly(ethylene terephthalate) (PET), polystyrene (PS), polycarbonate (PC), TAC (triacetyl cellulose), polyimide (PI), nylon (Ny), low density polyethylene (LDPE), medium density polyethylene (MDPE), poly(vinyl chloride), poly(vinylidene chloride), poly(phenylene sulfide), poly(ether sulfone), poly(ethylene naphthalate), polypropylene, and an acrylic material, such as poly(urethane acrylate). The silicone-based material comprise, for example, polydimethylsiloxane (PDMS). Examples of the inorganic materials comprise glass (including glass fiber), pottery (e.g. ceramics, and enamel), metal, a carbon fiber, and a calcium phosphate structure (e.g. bone, tooth, and shell).

The above materials may be used singly, or two or more kinds of organic materials, inorganic materials, or a combination of an organic material and an inorganic material may be used in combination. For example, there is a fiber reinforced plastic (FRP), which is a combination of carbon fibers or glass fibers and a synthetic resin.

There is no particular restriction on the size of the support material. It may be appropriately selected according to the purpose. There is no particular restriction on the shape of the support material, and it may be appropriately selected according to the purpose. For example, it may have a three-dimensional shape such as a dish, a multi-plate, a flask, a membrane, and a cell culture insert, or shape of a flat plate or flat membrane, such as a glass plate, a slide glass, and a cover glass.

There is no particular restriction on the structure of the support material, and it may be appropriately selected according to the purpose. Examples thereof comprise a porous structure, a mesh structure, a relief structure, and a honeycomb structure. Since a porous structure and a mesh structure can retain a large volume of solution, they are particularly preferable as the support material.

### 1-3. Production method

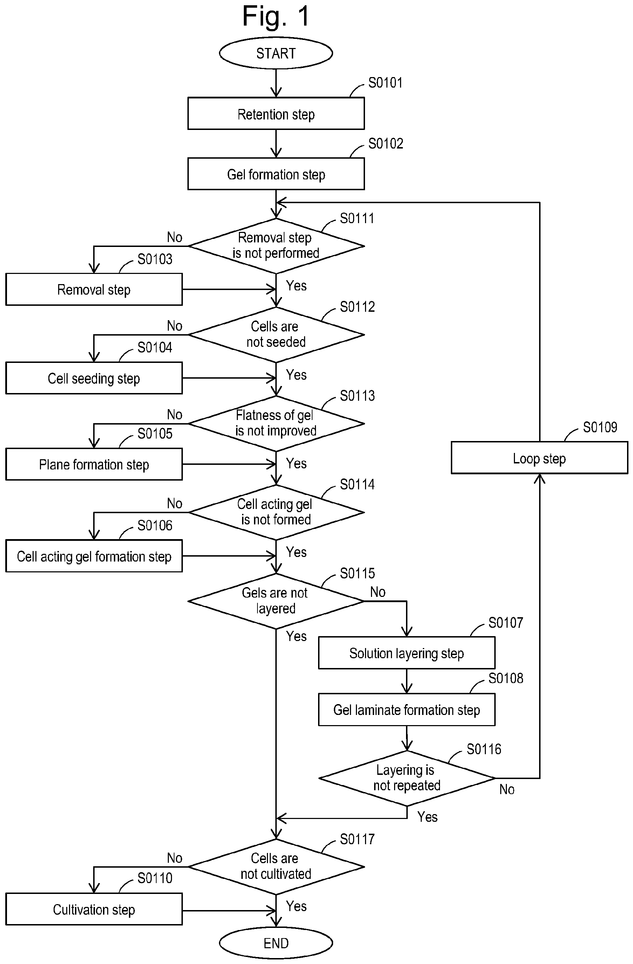

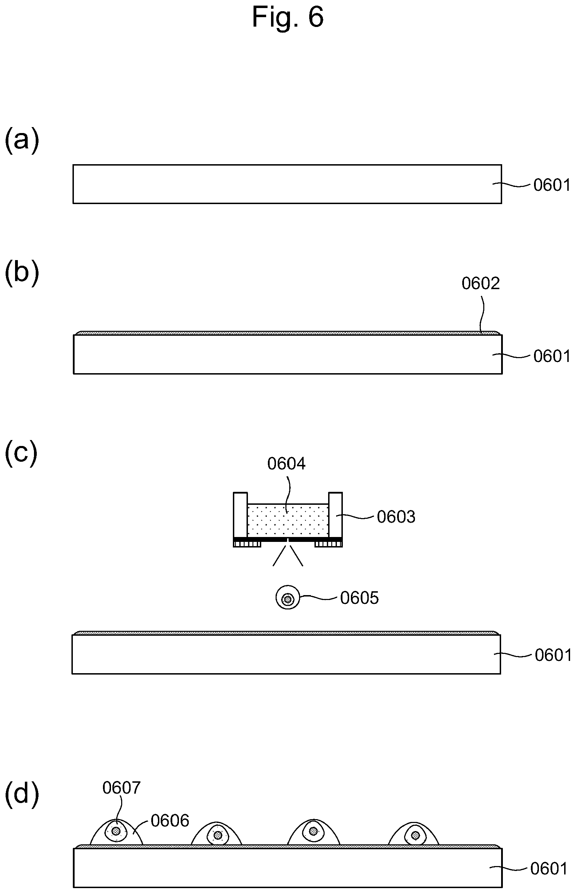

The process flow of the production method of the present invention is shown in Figure 1, and a schematic diagram of an example of the production process is depicted in Figure 6.

As shown in Figure 1, the production method of the present invention comprises as essential steps a retention step (S0101), and a gel formation step (S0102), and as optional steps a removal step (S0103), a plane formation step (S0104), a cell seeding step (S0105), a cell acting gel formation step (S0106), a solution stacking step (S0107), a gel stack formation step (S0108), a repeat step (S0109), and a cultivation step (S0110).

Further, in Figure 6, (a) to (d) represent a process chronologically until hydrogels (0606) containing cells (0607) are formed on a support material (0601). (a) depicts only the support material (0601), and (b) depicts a state where the support material (0601) retains the first solution (0602), which corresponds to the state after the retention step (S0101) in the production method of the present invention. (c) depicts a state where the second solution (0604) containing cells is discharged from a second solution discharge head (0603) to the support material (0601) in (b) in a form of a droplet (0605) such that the droplet lands on the support material. (d) depicts a state where a hydrogel (0606) is formed on the support material (0601) as a result of the landing of the second solution, which corresponds to the gel formation step (S0102) of the present invention.

The respective steps of the production method of the present invention will be specifically described below.

### (1) Retention step

The "retention step" (S0101) is a step of retaining the first solution. The retaining location is not limited. For example, it may be on a support material or on a hydrogel. The method for retaining the first solution is also not particularly restricted. For example, the support is dipped in the first solution so that the support material is impregnated with the first solution such that the first solution is retained in the entire surface and/or the inner part. Alternatively, the first solution may be discharged by a discharge method using a droplet discharge device, such as the inkjet method described in detail in the next step, so that the first solution is fixed and retained at a specific position on the support surface or the hydrogel surface. Further, as described above, the first solution may be retained using a cell layer as the support material.

When a cell layer is used as the support material, there is no particular restriction on the cell density on the support material in retaining the first solution, and it may be appropriately decided according to the use or purpose of the cell culture carrier. For example, it may be in a range of 1000 cells/cm² or more, 1500 cells/cm² or more, 2000 cells/cm² or more, 2500 cells/cm² or more, or 3000 cells/cm² or more, and 10000 cells/cm² or less, 9500 cells/cm² or less, 9000 cells/cm² or less, 8500 cells/cm² or less, 8000 cells/cm² or less, 7500 cells/cm² or less, or 7000cells/cm² or less.

### (2) Gel formation step

The "gel formation step" (S0103) means a step in which the second solution is shot by a droplet discharge device so as to land and come into contact with the first solution retained in the retention step (S0101), such that the first solution and the second solution are mixed to make the multiple branching polymers with polyethylene glycol as the backbone contained in the respective solutions react to form a hydrogel.

To "come into contact" means that the first solution and the second solution are mixed by the contact.

The "droplet discharge device" is means that ejects a solution stored in a liquid chamber as a droplet to land at the target site. Examples of the discharge method of the discharge device comprise an inkjet method, and a dispenser method by a gel extrusion method. By the inkjet method, the solution is ejected from a discharge hole (nozzle). Since the discharge method can discharge a minute solution (sometimes referred to as "droplet") from a discharge hole, it is possible to produce a highly accurate three-dimensional structure. At this time, the droplet discharged from the liquid discharge device may or may not contain cells.

The volume of a droplet to be discharged may be any liquid volume. Preferably, it is 9 pL or more, 15 pL or more, 20 pL or more, 30 pL or more, 40 pL or more, 50 pL or more, 60 pL or more, 70 pL or more, 80 pL or more, 90 pL or more, or 100 pL or more, and 900 pL or less, 800 pL or less, 700 pL or less, 600 pL or less, 500 pL or less, 400 pL or less, or 300 pL or less.

"Land" means that a solution is brought into contact with the target site, as shown in Figure 6 (c). This is achieved by discharging a droplet toward the target site according to the discharge method.

When the second solution contains cells, the cell content is preferably from 5 × 10⁵ cells/mL to 1 × 10⁸ cells/mL, and more preferably from 1 × 10⁶ cells/mL to 5 × 10⁷ cells/mL. When the cell density is lower than this content, it is difficult to form a hydrogel containing an appropriate cell count, and conversely when it is higher, the discharge of the solution by a discharge method such as the inkjet method becomes difficult.

The position where the solution is discharged is not particularly limited. It is only required to discharge the solution such that the solution lands at a desired target position. In addition, the respective landing sites may be apart, or partly in contact or overlapped

Since gel formation relies on a reaction between the multiple branching polymers that comprise polyethylene glycol as the backbone with a nucleophilic functional group and an electrophilic functional group, a hydrogel is formed when the second solution lands on the first solution. When the second solution is discharged with a droplet discharge device, a hydrogel may be formed usually but not exclusively as a dot-shaped hydrogel per single landing. "Dot-shaped" means herein a dot-like shape. Therefore, it is not limited to a perfect circle shape or a hemispherical shape, and it may be any shape comprising a nearly circular shape, or a nearly hemispherical shape, or further a polygonal shape, a nearly polygonal shape, an undefined shape, and a combination thereof. Further, there is no particular restriction on the dot-shape insofar as it has a predetermined length and thickness. In a case where the second solution is discharged multiple times, hydrogels with various shapes based on a dot-shaped hydrogel as the minimum unit can be produced.

The volume of a hydrogel, namely a dot-shaped hydrogel, formed by the crosslinking reaction caused by single landing depends on the number of discharge times of the second solution to the same site. In this regard, when the discharge hole diameter is larger, or the number of discharge times to the same site is larger, the volume of a dot-shaped hydrogel becomes larger. Therefore, the volume of a dot-shaped hydrogel can be regulated by changing the number of discharge times to the same site, or the discharge hole diameter. Herein, the volume of a dot-shaped hydrogel is preferably, but without limitation to, 9 pL or more, 15 pL or more, 20 pL or more, 30 pL or more, 40 pL or more, 50 pL or more, 60 or more, 70 pL or more, 80 pL or more, 90 pL or more, or 100 pL or more, and 900 pL or less, 800 pL or less, 700 pL or less, 600 pL or less, 500 pL or less, 400 pL or less, or 300 pL or less. The diameter of a dot-shaped hydrogel should be, but without limitation to, 10 µm or more and 300 µm or less, and the thickness should be 5 µm or more and 150 µm or less.

In this step, the second solution is discharged for an arbitrary number of times in one step, therefore a plurality of dot-shaped hydrogels may be formed. All or part of the dot-shaped hydrogels may be in contact with each other. When a plurality of dot-shaped hydrogels are placed in a row, it is possible to form not only dot-shaped but also optionally-shaped hydrogels. The shape can be appropriately selected according to the purpose. For example, by arranging dots in a uniaxial direction, a linear hydrogel can be formed. In addition, by arranging linear hydrogels in the same plane without gaps. a membranous (plane-shaped) hydrogel can be formed. By controlling the fusion and isolation among dot-shaped hydrogels to form linear or membranous hydrogels, the migration and extension of cells contained in each dot-shaped hydrogel can be regulated or suppressed. Furthermore, by forming membranous hydrogels, the gels can be easily stacked.

When cells are added into the solution, since the number of cells contained in a dot-shaped hydrogel is dependent on the supplied concentration, the cell density can be regulated by the number of formation times of dot-shaped hydrogels.

### (3) Removal step

The "removal step" (S0103) is a step of removing unreacted, namely not crosslinked portion of the multiple branching polymer existing, for example, on the support material or the hydrogel after the gel formation step (S0103), and before the solution stacking step (S0106) to be described later. This step is an optional step (S0111), and may be performed as needed.

The removal method is not particularly limited. Any of the publicly known removal methods may be used insofar as the method does not have a physical, biological, or chemical effect on the formed hydrogel. A simple removal method that is usually performed is to wash the support material or the hydrogel with an appropriate cleaning liquid.

There is no particular restriction on the cleaning liquid used in the cleaning method, insofar as it is a solution that does not affect hydrogels and cells. It may be selected as appropriate considering the pH, osmotic pressure, etc. Preferable examples comprise a buffer solution and a culture medium.

As for the cleaning method, the cleaning liquid may be poured on a support material or a hydrogel, or the object may be dipped in the cleaning liquid for the sake of alleviation of physical damage to the hydrogel. Cleaning may be performed multiple times in one step.

### (4) Plane formation step

The "plane formation step" (S0104) is a step of forming a hydrogel layer by stacking the first solution and the second solution on the hydrogel formed in the gel formation step (S0102) or the gel stack formation step (S0108) to be described later, and the support material, or the cell acting gel formed in the cell acting gel formation step (S0106) to be described later. This step is an optional step (S0112), and may be performed as needed.

There may be a difference in height between the hydrogel formed in the gel formation step (S0102) or the gel stack formation step (S0108), or the cell acting gel formed in the cell acting gel formation step (S0106) and the surroundings, because a dot-shaped hydrogel, or a dot-shaped cell acting gel as the constituting unit has an arbitrary thickness. In this step, such a difference in height between the hydrogels and the surroundings is reduced by filling the surroundings of the dot-shaped hydrogel or cell acting gel with hydrogels. When gels are stacked, another object is to improve the flatness of the lower gel layer serving as the substrate of the stack. In addition, since mixing between layers is suppressed, there occurs no contamination of cells or materials in the adjacent layer.

The basic operation of this step may be the same as the retention step (S0101) or the gel formation step (S0102). However, the first solution and the second solution used in this step may or may not contain cells.

### (5) Cell seeding step

The "cell seeding step" (S0104) is a step of seeding cells on the formed hydrogels. This step is an optional step (S0113), and may be carried out as necessary. Further, the order of this step, the removal step (S0103), and the plane formation step (S0105) does not matter insofar as they are performed after the gel formation step (S0102). The cell seeding step (S0104) may be performed after the removal step (S0103) and the plane formation step (S0105), or the removal step (S0103) and the plane formation step (S0105) may be performed after the cell seeding step (S0104).

The hydrogel produced by this production method contains a cell acting additive as necessary. By seeding cells to a hydrogel containing a cell acting additive, the cells can be adhered to the hydrogel. In addition, by seeding cells to the cell acting gel described later, cell patterning outside the gel can be performed.

There is no particular restriction on the cell seeding method, and it can be appropriately selected according to the purpose. Example thereof comprise a method in which a solution containing cells is discharged by the droplet discharge device such that the cells are fixed and retained at a specific position on a hydrogel, or a cell acting gel. At this time, the cell content per unit volume of solution is preferably from 5 × 10⁵ cells/mL to 1 × 10⁸ cells/mL, and more preferably from 1 × 10⁶ cells/mL to 5 × 10⁷ cells/mL.

### (6) Cell acting gel formation step

The "cell acting gel formation step" (S0106) is a step of stacking a cell acting gel on a support material or a hydrogel. This step is an optional step (S0114), and may be carried out as necessary. The cell acting gel contains a cell acting additive as the main gel component instead of a multiple branching polymer. It may further contain various components other than the multiple branching polymer. For example, it may comprise a dispersion medium, cells, or a combination thereof.

This step can be performed after the gel formation step (S0102). It is preferably performed after the plane formation step (S0105) or the cell seeding step (S0104).

As the gel formation method in the cell acting gel formation step (S0106), a method by which a cell acting gel is discharged by a droplet discharge device is preferable, but not limited thereto. In this case, the specific method conforms to the aforementioned gel formation step (S0102). A cell acting gel to be formed by a single discharge will present a dot shape similar to the dot-shaped hydrogel. Such a cell acting gel is referred to herein as "dot-shaped cell acting gel".

### (7) Solution stacking step

The "solution stacking step" (S0107) is a step of stacking again the first solution on the formed hydrogel or cell acting gel. Although this step is an optional step (S0115), it is an essential step in a case in which hydrogels are formed into a stack.

This step and the gel stack formation step (S0108) to be described later aim to layer a new hydrogel on the hydrogel layer that has already been formed.

The basic operation of this step conforms to the retention step (S0101). In this step, the first solution is stacked such that the first solution is retained on all or part of the formed hydrogel or cell acting gel. At this time, for example, the first solution may be retained again on the support material on which a hydrogel has not yet been formed.

There is no particular restriction on the method for retaining a hydrogel, and it may be appropriately selected out of the publicly known methods according to the purpose. Examples thereof comprise a method by which a support material where a hydrogel has been formed is immersed in the first solution to impregnate the surface of the hydrogel with the first solution identically with the aforementioned method for retaining the first solution, and a method by which the first solution is discharged by a droplet discharge device, so that the first solution is fixed and retained at a specific position on the hydrogel.

The first solution used in this step may be the same as, or different from the first solution used before this step in the production method of the present invention. For example, it may contain a different kind of dispersion medium than the dispersion medium used in the already formed hydrogel.

### (8) Gel stack formation step

The "gel stack formation step" (S0108) is a step of stacking a new hydrogel on a hydrogel or a cell acting gel by discharging the second solution to land such that the second solution comes into contact with the first solution stacked on the hydrogel or cell acting gel in the solution stacking step (S0107). Although this step is an optional step, it is an essential step in a case in which hydrogels are formed into a stack.

This step is similar to the gel formation step (S0102). In this step, the position where the second solution is discharged is not particularly limited. It is required, however, that at least a part of the second solution discharged should land on all or part of the hydrogel or the cell acting gel. When discharge is performed for multiple times, all or part of dot-shaped hydrogels formed on the hydrogel or the cell acting gel may be in contact with each other as in the gel formation step (S0102). When a plurality of dot-shaped hydrogels are placed in a row on a hydrogel-cell composite, a thick multi-stacked hydrogel can be formed.

The second solution to be used in this step may be the same as, or different from the second solution used before this step in the production method of the present invention. For example, it may contain cells of a different kind from the cells used in the already formed hydrogel.

### (9) Repeat step

The "repeat step" (S0109) is a step in which the removal step (S0103) to the gel stack formation step (S0108) are repeated for a plurality of times. Although this step is an optional step (S0116), it should be preferably carried out when a three-dimensional hydrogel with a sufficient height is to be formed.

In implementing this step, the removal step (S0103), the cell seeding step (S0105), and the plane formation step (S0104) can be carried out as needed.

This step aims at formation of a multi-stacked hydrogel. By this step, a steric (three-dimensional) hydrogel can be produced.

### (10) Cultivation step

The "cultivation step" (S0110) is a cultivation step of culturing cells that are contained in the formed hydrogel, and/or cells in contact with a hydrogel and/or a cell acting gel. This step is an optional step (S0117), and may be carried out as necessary.

The hydrogel produced by this production method optionally contains living cells in the gel depending on the purpose. When the hydrogel contains a culture medium as a dispersion medium, the cells in the gel can be retained for a certain period of time. However, when the hydrogel is stored, or the cells in the gel are grown or extended, it is necessary to retain the cells in the gel for a long period of time. In this case, the management of additional nutrient supply to the cells, gas exchange, waste product removal, etc. becomes required. The requirement can be met by culturing the cells that are contained in, and/or the cells in contact with the hydrogel formed in this step by the production method of the present invention. There is no particular restriction on the cell cultivation method, insofar as it can cultivate the cells that are contained in, and/or the cells in contact with the hydrogel. Examples thereof comprise a method in which culture is performed by dipping the hydrogel in a culture medium.

A culture medium may be appropriately selected out of the culture media publicly known in the art as described in the section of "1-2. Definition of terms" according to the purpose or cell type.

The culture method may be the same as ordinary cell culture methods, and there is no particular limitation. For example, a hydrogel may be dipped in a culture tank containing a culture medium, and cultured in an environment of 5 volume-% CO₂ at 37°C. The culture medium may be exchanged at intervals of several days as appropriate depending on the proliferation status of cells, etc., or the culture medium may be mobilized to supply sufficient oxygen and nutrients to each cell. For mobilizing the culture medium, stirring using a stirrer or a stirring rod, shaking of the culture tank, or circulation using a peristaltic pump, or the like may be performed. However, the mobilization of the culture medium should preferably be carried out gently to avoid any physical damage to the hydrogel due to the flowing water pressure, etc.

The culture period is not particularly limited. The cultivation may be carried out for an appropriate period as needed. Usually, one day to 30 days are enough. It is preferably 1 day to 7 days.

### 2. Cell culture carrier

### 2-1. Outline

A second aspect of the present invention is a cell culture carrier. The cell culture carrier of the present invention is constituted with hydrogels comprising a multiple branching polymer, and may be formed into an optional shape, such as dot-shaped, linear, membranous, or three-dimensional shape. It is possible that the cell culture carrier of the present invention provides a three-dimensional structure with high modeling reproducibility and shape retainability. Further, it can provide an artificially formed three-dimensional structure capable of cultivating the cells inside the gel, or on the gel surface for a long period of time. The cell culture carrier of the present invention can be produced by the production method for a cell culture carrier described in the first aspect.

### 2-2. Constitution

The hydrogel constituting a cell culture carrier of the present invention is composed of one or more dot-shaped hydrogels. The "dot-shaped hydrogel" is the constituent unit of the hydrogel in the cell culture carrier of this aspect. As described in the first aspect, when the second solution is discharged by the discharge method to land on the first solution, a hydrogel formed per single discharge is called dot-shaped hydrogel.

The shape, size, thickness, etc. of the dot-shaped hydrogel constituting the cell culture carrier of this aspect are similar to those in the first aspect.

The dot-shaped hydrogel may contain cells, a dispersion medium, or an additive (*e.g.*, cell acting additive). In this case, the cells, dispersion medium, and additive to be contained in the respective dot-shaped hydrogels may be the same or different kinds.

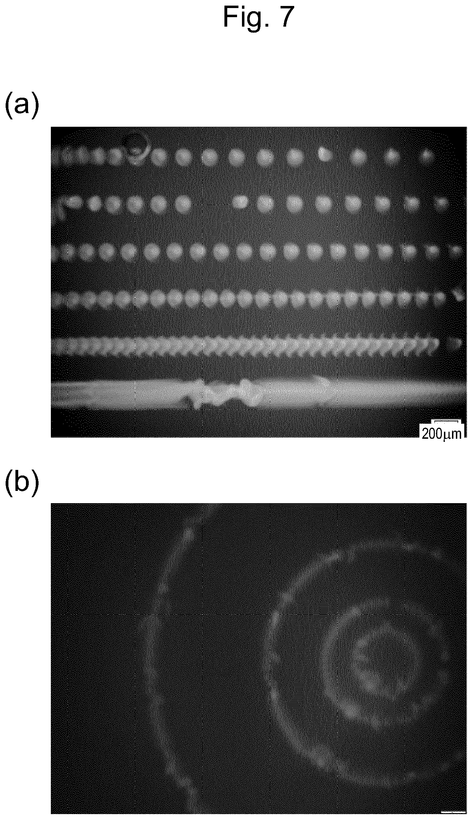

The cell culture carrier of the present invention has a point-like structure constituted with a single dot-shaped hydrogel, or with a plurality of mutually independent dot-shaped hydrogels, and a linear structure and/or membranous structure formed with such hydrogels in contact with each other. It may also have a three-dimensional structure in which a plurality of hydrogels are stacked on the dot-shaped hydrogels with a point-like structure and/or linear structure and membranous structure such that all or part of them overlap each other. Figure 7 shows examples of a hydrogel which is formed by controlling contiguity of dot-shaped hydrogels with a solution discharge head whose discharge velocity is regulated. (a) shows a patterning of dot-shaped hydrogels connected linearly by decreasing little by little the pitches between dot-shaped hydrogels. (b) shows a patterning of dot-shaped hydrogels into a curved linear shape formed by connecting dots on a concentric circumference.

After gel formation, the support material may remain as it is, or may be removed. In the latter case, various evaluations may be performed using only a cell culture carrier.

In the present invention, the "shape retainability" refers to a property concerning the temporal change of the shape. When the shape retainability of a substance is high, it means that the temporal change of the substance is small, and the shape at the time of production is prone to be maintained. The cell culture carrier of the present invention comprises a hydrogel, and the hydrogel has a high shape retention rate. The "shape retention rate" herein is the rates of change in the diameter and thickness of the dot-shaped hydrogel, which is cultured for 3 days after being placed in an incubator immediately after the hydrogel preparation. In the case of the cell culture carrier hereunder, the shape retention rate concerning the diameter and thickness of the hydrogel is 75% or higher. With respect to a linear hydrogel it is evaluated similarly based on the line width and thickness, and with respect to a membranous hydrogel it is evaluated based on the membrane thickness.

### 3. Production device for cell culture carrier

### 3-1. Outline

A third aspect of the present invention is production device for a cell culture carrier. The production device of the present invention is the device that embodies the production method for a cell culture carrier described in the first aspect, and capable of producing a cell culture carrier comprising a hydrogel with high modeling reproducibility and shape retainability. Further it can produce a cell culture carrier comprising a hydrogel that has an optional shape and is capable of culturing the cells in a gel for a long period of time.

### 3-2. Constitution

The production device for a cell culture carrier of the present invention comprises at least stage means of fixing a substrate, retention means of retaining a first solution, first solution discharge means of discharging the first solution to a substrate placed on the stage means, retention means of retaining a second solution, second solution discharge means of discharging the second solution to a substrate placed on the stage means, solution discharge control means of controlling the discharge of the respective solutions in the first solution discharge means and the second solution discharge means, discharge position control means of controlling the discharge positions of the respective solutions by calculating the relative positions of the first solution discharge means and the second solution discharge means, and the stage, and controlling the discharge positions of the respective solutions, and a discharge order control means of controlling the discharge order of the first solution discharge means and the second solution discharge means. It may further comprise means of removing an unreacted multiple branching polymer with polyethylene glycol as the backbone remaining in the surface of the substrate on which a gel is formed. The respective parts and means will be described below.

### (1) Stage means

The "stage means" is a part for fixing a substrate in production device for a cell culture carrier. The stage means is so configured to fix the substrate at the right position.

The "substrate" in the production device of this aspect is a member that is placed on the stage means, and captures the solutions discharged from the first solution discharge means or the second solution discharge means at a predetermined position on its surface to form a hydrogel. Examples thereof comprise a support material, and a hydrogel. In a case where hydrogels are stacked by the production device of this aspect it is necessary to place a solution containing a gelling material accurately on a hydrogel which constitutes the substrate, and therefore it is preferable that positioning of the substrate is performed.

There is no particular restriction on the fixing method for a substrate, insofar as a substrate can be fixed on the stage means, and a publicly known method may be also applicable. For example, a substrate may be fixed by pressing it from the top onto the stage means, or pinching it from the side using a fixing means such as a spring clip fixed on the stage means,

### (2) Retention means

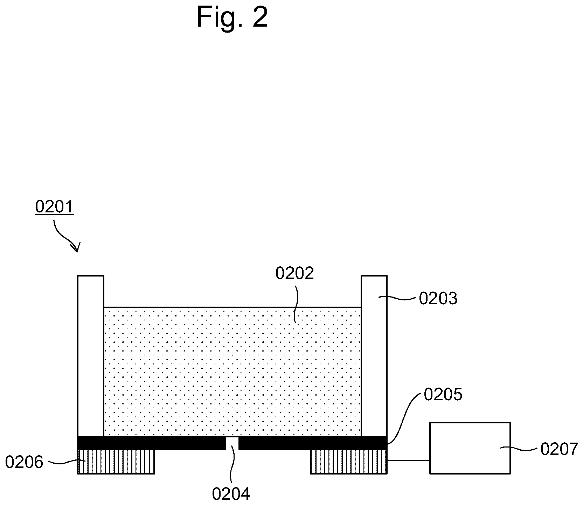

The "first solution retention means" and the "second solution retention means" are the means configured such that the respective solutions are supplied to the "first solution discharge means" and the "second solution discharge means" to be discharged. The basic configurations for the first solution retention means, and the second solution retention means are the same, except that the former retains the first solution and the latter retains the second solution. There is no particular restriction on the material quality or shape, insofar as the first solution or the second solution can be retained, and a publicly known method may be applied. For example, in the case of an inkjet head for solution discharge (herein often referred to as "solution discharge head") as depicted in the schematic diagram in Figure 2, a liquid can be retained because the solution discharge head (0201) is provided with a liquid chamber (0203) for accommodating a liquid (0202).

### (3) First and second solution discharge means

The "first solution discharge means" and the "second solution discharge means" (hereinafter the two parts are collectively referred to as "solution discharge means") are means which are so configured that they can discharge the respective solutions onto a substrate placed on the stage means.

The basic configurations for the first solution discharge means, and the second solution discharge means are the same, except that the former discharges the first solution and the latter discharges the second solution.

Specific configuration example of the solution discharge means comprise an inkjet head. Figure 2 is a schematic diagram of an inkjet head for solution discharge. As shown in this figure, the solution discharge head (0201) is provided with a liquid chamber (0203) for accommodating a liquid (0202), a membrane (0205) in which a discharge hole (nozzle) (0204) is formed, and a vibration device (0206). In this figure, a driving means (0207) is also shown, which applies a voltage to the vibration device (0206) as a specific driving signal, and corresponds to a solution discharge control means described later.

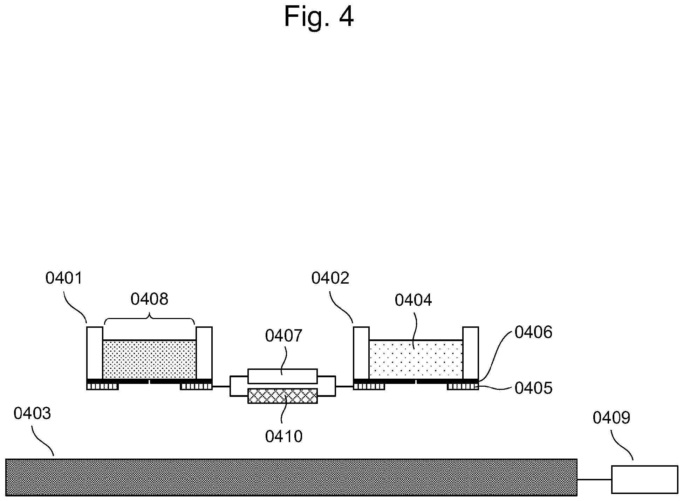

The production device of the present invention may respectively comprise a plurality of first solution discharge means and second solution discharge means. In that case, each solution discharge means may retain the same or different solutions. For example, Figure 4 shows an example of production device provided with two second solution discharge means (second solution discharge heads). At this time, there is a case where the second solution discharge head 1 (0401) and the second solution discharge head 2 (0402) comprise mutually different cells. In Figure 4, 0403 stands for the stage, 0404 stands for the liquid chamber unit, 0405 stands for the vibration unit, 0406 stands for the membranous member, and 0407 stands for the driving unit, 0408 stands for the atmospheric vent unit, 0409 stands for the stage driving means, and 0410 stands for the signal control means.

### (4) Solution discharge control means

The "solution discharge control means" is means that controls discharge of each solution in a solution discharge means. With the solution discharge control means, a solution discharge signal is transmitted to each solution discharge means to regulate the discharge timing, number of discharge times, or discharge volume of the solution. As a specific configuration example of the solution discharge control means, there is a driving means (0207) shown in the Figure 2.

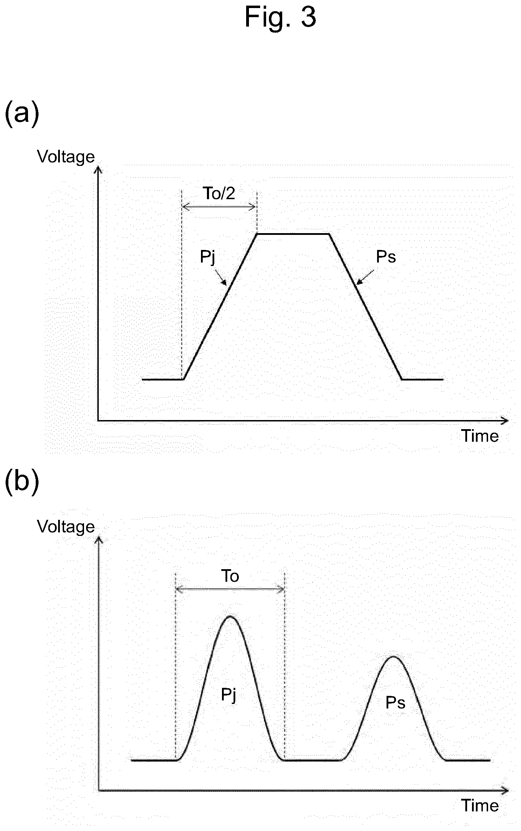

Figure 3 shows an example of the input waveform from the driving means (0207) to the solution discharge head. The driving means (0207) is so configured to transmit the discharge waveform (Pj) presented in Figure 3 (a) as the driving signal to the vibration device (0206) of the inkjet head presented in Figure 2 to regulate the vibration condition of the membrane (0205) via the vibration device (0206) to discharge the liquid (0202) retained in the liquid chamber (0203), namely the solution, in a form of droplets. The discharge waveform (Pj) is preferably set at a driving signal comprising the proper oscillation period (Tₒ) of the membrane as shown in Figure 3 (b) in order to discharge a liquid at the lowest possible voltage by causing sympathetic vibration of the membrane. For the discharge waveform (Pj), not only a triangular wave and a sine wave, but also a triangular wave whose edge is made gentle by applying a low-pass filter can be used.

Further, the driving means (0207) is so configured that an attenuation waveform (Ps) presented in Figure 3 (a) can be transmitted as a driving signal to the vibration device (0206). By this driving signal, the membrane residual vibration after droplet formation may be suppressed quickly, so that the higher frequency consecutive discharges become possible. In addition, since satellites and mists are reduced, finer liquid volume regulation becomes possible. For the attenuation waveform (Ps), not only a triangular wave and a sine wave, but also a triangular wave whose edge is made gentle by applying a low-pass filter can be used.

There is no particular restriction on the volume of each solution retained in the liquid chamber (0203) of the solution discharge head (0201), and the liquid chamber is typically so configured to retain the volume of about 1 µL to 1 mL. In particular, when the solution itself is expensive, such as a cell suspension in which cells are dispersed, the solution discharge head is preferably so configured that a droplet can be formed with a small volume of liquid. In this regard the liquid chamber (0203) may be so configured to retain a liquid in an volume of about 1 µL to 200 µL.

The shape of the membrane (0205) in a plan view is not particularly limited. For example, any of a circular, oval, or square shape can be used. The shape conforming to the shape of the bottom of the liquid chamber (0203) is preferable. Meanwhile, although there is no particular restriction on the material quality of the membrane (0205), the membrane with a too soft material is prone to vibrate even when a solution is not discharged, and it is difficult to suppress vibrations. Therefore, a material with a certain degree of hardness is preferable. Generally, metal, ceramic, or a polymer material such as a plastic with a certain degree of hardness is used. In the case of metal, for example, but not limited to, stainless steel, nickel, aluminum, or the like may be used. In the case of ceramic, for example, but not limited to, silicon dioxide, alumina, zirconia, or the like may be used.

It is desirable that the discharge hole (0204) is formed in the center of the membrane (0205) as a substantially perfectly circular through hole.

As a specific example of the vibration device (0206) there is a piezoelectric device. By applying a voltage to the piezoelectric device, a compressive stress acts in the lateral direction of the page, so that the membrane can be deformed. As the material of the piezoelectric device (piezoelectric material), lead zirconate titanate, which is most popular material in the art, may be used. Alternatively, bismuth ferric oxide, metal niobate, barium titanate, or these materials to which a metal or a different oxide is added, may be used as a piezoelectric material. As another example besides a piezoelectric device, there is a device in which a material having a coefficient of linear expansion different from that of the membrane is bonded to the membrane. This device can deform the membrane utilizing the difference in coefficient of linear expansion when heated. The device is preferably so configured that a heater is installed on the materials having different coefficients of linear expansion, and when the heater is energized the membrane is deformed to discharge droplets.

### (5) Discharge position control means

The "discharge position control means" is means for controlling the discharge position of each solution. This means is configured such that the discharge position of the solution from the solution discharge means can be regulated by calculating the relative position of the solution discharge means and the stage, in order to land the solution at a specific position on the substrate fixed on the stage. By the signal from the discharge position control means, the solution discharge means or the stage is moved so that the discharge hole can be positioned at a predetermined position on the substrate where the solution should be discharged. Alternatively, it may be so configured that the direction of the discharge hole can be regulated. Specific examples of the configuration of the discharge position control means comprise the stage driving means (0409) presented in Figure 4 as described above.

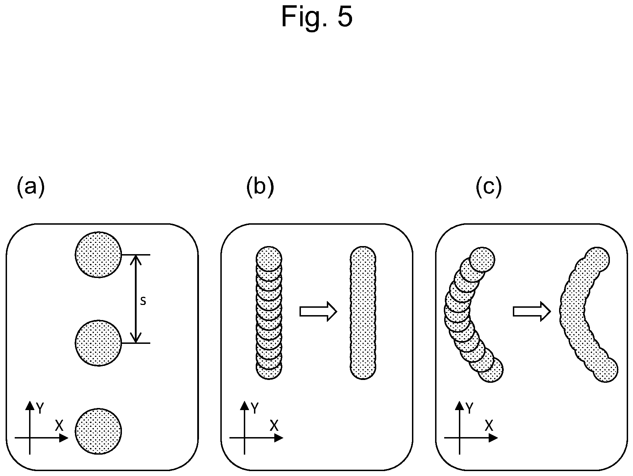

By instructing the position where the hydrogel should be formed on the substrate to this means, a hydrogel of an optional shape can be formed on the substrate. Figure 5 is schematic diagrams of hydrogels formed in various shapes on the support material by this means. In Figure 5 (a), dot-shaped hydrogels are formed to have a pitch (S). In Figure 5 (b), dot-shaped hydrogels are in contact with each other and contiguously overlapped to form a linear non-individual body (linear hydrogel). In Figure 5 (c), dot-shaped hydrogels are similarly overlapped but in a curve to form a curved linear non-individual body (linear hydrogel). In this regard, fusion by connection of dot-shaped hydrogels can be achieved by regulating the discharge velocity of the solution by the solution discharge control means. For example, by discharging the second solution to the next predetermined position after discharge of the second solution but before the gelation between the first solution and the second solution is completed, dot-shaped hydrogel fusion linear non-individual body (linear hydrogel) as depicted on the right side of Figure 5 (b) or 5 (c) can be obtained.

### (6) Discharge order control means

The "discharge order control means" is means that controls the discharge order of the solutions from the first and the second solution discharge means. This means transmits a control signal to the solution discharge control means to control the order of solution discharges from the respective solution discharge mean. By this means, the steps in the production method according to the first aspect can be executed. Examples of a specific configuration of the discharge order control means comprise the signal control means (0410) shown in Figure 4 described above.

### (7) Removal means

The "removal means" is means for removing an unreacted multiple branching polymer with polyethylene glycol as the backbone remaining on the support material on which gels are formed, and on the hydrogel. By this means, the removal step in the production method according to the first aspect can be carried out.

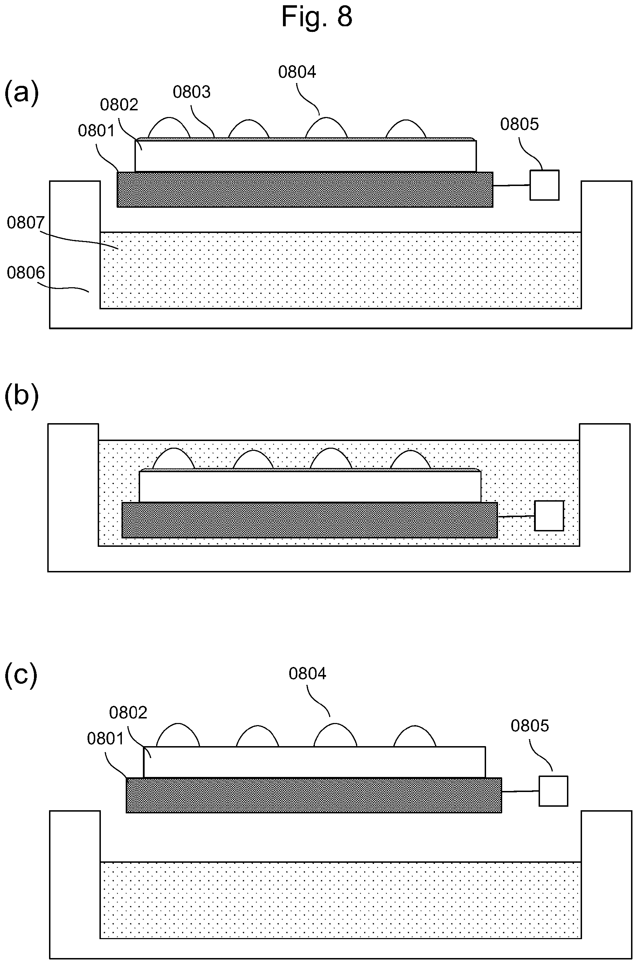

As a specific example, Figure 8 presents schematic diagrams of the removal means. The dot-shaped hydrogels (0804) formed on the support material (0803) on which the unreacted multiple branching polymer (0802) with polyethylene glycol as the backbone remains, and which is on the stage (0801), are placed by a stage driving means (0805) in the removal means configured as a cleaning vat (0807) retaining inside a cleaning liquid (0806). As shown in Figure 8 (b), the support material is immersed in the cleaning liquid in the cleaning vat for washing, so that a cell culture carrier from which the unreacted multiple branching polymer with polyethylene glycol as the backbone has been removed can be obtained as shown in Figure 8 (c).

### 4. Method for using cell culture carrier

A fourth aspect of the present invention is a method for using a cell culture carrier. A cell culture product can be yielded by cultivating cells using a cell culture carrier produced by the production method for a cell culture carrier described in the first aspect. The "cell to be cultured" may be a cell seeded or embedded in a cell culture carrier produced by the production method described in the first aspect. Meanwhile, the cell comprised in the cell culture carrier produced by the production method described in the first aspect may be an accessory cell such as a feeder cell, while the "cell to be cultured" may be a cell to be seeded onto a cell culture carrier containing an accessory cell. The "cell to be cultured" may also be a cell comprised in a droplet discharged from the droplet discharge device in the (2) gel formation step according to the first aspect, or a cell to be seeded in the (5) cell seeding step according to the first aspect and to be comprised in the production process of the cell culture carrier.

### Examples

The present invention will be specifically described below with reference to Examples, provided that the present invention is not restricted by the following Examples.

### (Method)

### <Example 1>

In this example, a cell culture carrier in which dot-shaped hydrogels were formed on the support material was prepared using the production method for a cell culture carrier of the present invention.

### (1) Preparation of first solution

The first solution containing 2% tetra-PEG-SH was prepared by dissolving tetra-PEG-SH (trade name: SUNBRIGHT PTE-100SH, produced by Yuka Sangyo Co., Ltd.) in PBS(-) (Thermo Fisher Scientific), and then filtrating the solution through a filter with an average pore diameter of 0.2 µm (trade name: Minisart Syringe Filter 175497K, produced by Sartorius).

### (2) Preparation of second solution

The second solution containing 2% tetra-PEG- maleimidyl was prepared by dissolving 0.1g of tetra-PEG-maleimidyl (trade name: SUNBRIGHT PTE-100MA, produced by Yuka Sangyo Co., Ltd.) in PBS(-), and then filtrating the solution through a filter with an average pore diameter of 0.2 µm.

### (3) Preparation of support material

A polyester-made porous culture membrane with a diameter of 13 mm (trade name: ipCELLCULTURE Track-Etched Membrane Filter, pore size: 0.45 µm, pore density: 4E6 cm⁻², thickness: 12 µm, produced by it4ip) was placed in a 35 mm dish, to which 2 mL of the first solution was added to impregnate the membrane with the first solution. Around the center of an 18 mm-square cover glass (grade name: No.1, Thickness: from 0.13 to 0.17 mm, produced by Matsunami Glass Ind., Ltd.), 3 µL of the second solution was placed and the membrane impregnated with the first solution was stacked on the second solution part to perform adhesion by means of a hydrogel, such that a hydrogel was formed with tetra-PEG-SH and tetra-PEG-maleimidyl, which fixed the membrane to the cover glass. The cover glass with the fixed membrane was placed in a dish containing the first solution, so that the membrane was impregnated again with the first solution. Then, it was taken out from the dish to obtain a support material retaining the first solution.

### (4) Hydrogel formation

After filling the liquid chamber of the inkjet head for discharging the second solution (second solution discharge head) with the second solution, this solution was dropped onto the support one by one at intervals of 400 µm of 20 × 20 pitch so that the second solution landed on the first solution, and a hydrogel composed of a tetra-PEG gel was formed by the reaction of the first solution and the second solution. After formation, the hydrogel was transferred to a 35 mm dish, to which a 10 mass% FBS and a DMEM containing 1 mass% of an antibiotic were gently added, and the mixture was placed in the incubator (described above) at 37°C in an environment of 5 volume% CO₂.

### <Example 2>

In this Example, a cell culture carrier in which dot-shaped hydrogels containing cells were formed on the support material was produced using the production method for a cell culture carrier of the present invention. The basic procedure of this Example was the same as in Example 1. However, this Example was different from Example 1 in that a cell-containing second solution containing cells was used as the second solution.

### (1) Culture of cells

Normal human dermal fibroblasts (trade name: CC2507, produced by Lonza, hereinafter referred to as "NHDF cells") were cultured in a 100 mm dish for 72 hours in an incubator (trade name: KM-CC17RU2, manufactured by Panasonic Corporation, 37°C, environment of 5 volume% CO₂) and using a Dulbecco's Modified Eagle's Medium (trade name: DMEM (1×), produced by Thermo Fisher Scientific, hereinafter referred to as "DMEM") containing 10 mass% fetal bovine serum (hereinafter referred to as "FBS") and a 1 mass% antibiotic solution (Antibiotic-Antimycotic Mixed Stock Solution (100×), produced by Nacalai Tesque, Inc.).

### (2) Preparation of cell suspension

After culturing NHDF cells for 72 hours, DMEM in the dish was removed using an aspirator. To the dish 5 mL of PBS(-) was added, and the PBS(-) was removed by suction with an aspirator, and the surface was washed. After repeating twice the washing operation with PBS(-), 2 mL of a 0.05 mass% trypsin/0.05 mass% EDTA solution (produced by Thermo Fisher Scientific) was added to the dish, and heated in the incubator for 5 min to detach the cells from the dish. After confirming the detachment of cells by a phase-contrast microscope (device name: CKX41, manufactured by Olympus), 4 mL of an FBS-containing DMEM was added to the dish to deactivate trypsin. The cell suspension in the dish was transferred to a 15 mL centrifuge tube, subjected to centrifugation (trade name: H-19FM, manufactured by KOKUSAN Co. Ltd., 1.2 × 10³ rpm, 5 min, 5°C), and the supernatant was removed using an aspirator. After the removal, 2 mL of FBS-containing DMEM was added to the centrifuge tube, and the cells were dispersed by gentle pipetting to prepare a cell suspension. From the cell suspension, 20 µL was transferred to an Eppendorf tube, to which 20 µL of a 0.4 mass% trypan blue stain was added followed by pipetting. From the stained cell suspension, 20 µL was taken out and put on a PMMA-made plastic slide, and the cell count was counted using a cell counter (trade name: Countess Automated Cell Counter, manufactured by Thermo Fisher Scientific) and the cell count in the solution was calculated.

### (3) Preparation of second solution in which cells are suspended

Part of the cell suspension obtained in (2) was transferred to an Eppendorf tube and centrifuged (device name: MiniSpin, manufactured by Eppendorf AG, 2.5 × 10³ rpm, 1 min), and then the supernatant was removed using a pipette. After the removal, the second solution prepared in "Example 1-(2)" was added to obtain a cell-containing second solution which was composed of a cell suspension with the cell concentration of 1 × 10⁷ cells/mL containing tetra-PEG- maleimidyl.

### <Example 3>

In this Example, a cell culture carrier in which dot-shaped hydrogels containing cells were formed on the support material was produced using the production method for a cell culture carrier of the present invention. The basic procedure of this Example was the same as in Example 2. However, this Example was different from Example 2 in that the hydrogels were stacked by implementing the solution stacking step and the gel stack formation step in the production method.

### (1) Gel formation step, solution stacking step, and gel stack formation step

In the gel formation step, a hydrogel of 10 mm × 20 mm was formed by dropping droplets one by one at 100 µm pitch onto a support material by a second solution discharge head filled with a cell-containing second solution.

Next, as the solution stacking step, the hydrogels were placed into the dish containing the first solution, and impregnated with the first solution. Then, as the solution stacking step, the hydrogels were placed into the dish containing the first solution, and impregnated with the first solution.

Subsequently, as the gel stack formation step, hydrogels of the second layer were formed by dropping droplets one by one at 400 µm pitch onto the hydrogels by a second solution discharge head filled with a cell-containing second solution.

Finally, the stack was transferred to a 35 mm dish containing 2 mL of DMEM containing a 10 mass% FBS and a 1 mass% antibiotic, and placed in the incubator (described above) at 37°C in an environment of 5 volume% CO₂.

In addition, the cells in the first layer were stained with a green fluorescent dye (trade name: Cell Tracker Green, produced by Thermo Fisher Scientific), and the cells in the second layer were stained with an orange fluorescent dye (trade name: Cell Tracker Orange, produced by Thermo Fisher Scientific). Each was placed into the liquid chamber of the second solution discharge head, and discharged from the second solution discharge head to form a hydrogel. One hour after the preparation of the hydrogel, the hydrogel was observed with a confocal microscope FV10 (described above). The observation results are presented in Figure 9.

### <Example 4>

In this Example, a cell culture carrier in which a hydrogel containing cells was stacked on the support material was produced using the production method for a cell culture carrier of the present invention. The basic procedure of this Example was the same as in Example 3. However, in this example, the shape of the formed hydrogel was different from that in Example 3. That is, in Example 3, dot-shaped hydrogels were formed in the second layer, meanwhile in this Example, linear hydrogels were formed in the second layer.

### (1) Gel formation step, solution stacking step, and gel stack formation step

In the gel formation step, a hydrogel of 10 mm × 20 mm was formed by dropping droplets one by one at 100 µm pitch onto a support material by a second solution discharge head filled with a cell-containing second solution. Then, in the solution stacking step, the hydrogel was placed into the dish containing the first solution, and impregnated with the first solution.

Subsequently, as the gel stack formation step, 25 linear pattern hydrogels with a width of 200 µm and a length of 20 mm were formed at 400 µm intervals by dropping droplets one by one at 50 µm pitch onto the hydrogel by a second solution discharge head filled with a cell-containing second solution, with which the hydrogels of the second layer were formed.

Finally, the stack was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 5>

In this Example, a cell culture carrier in which a hydrogel containing cells was stacked on the support material was produced using the production method for a cell culture carrier of the present invention. The basic procedure of this Example was the same as in Example 4. However, this Example was different from Example 4 in that the repeat step was performed to repeat multiple times the solution stacking step and the gel stack formation step in the production method to stack a plurality of hydrogel layers.

### (1) Gel formation step, and solution stacking step and gel stack formation step comprising repeat step

As the gel formation step, a hydrogel of 10 mm × 20 mm was formed by dropping droplets one by one at 100 µm pitch onto a support material by a second solution discharge head filled with a cell-containing second solution. Then, as the solution stacking step, the hydrogel was placed into the dish containing the first solution, and impregnated with the first solution.

Subsequently, as the gel stack formation step, 25 linear pattern hydrogels with a width of 200 µm and a length of 20 mm were formed at 400 µm intervals by dropping droplets one by one at 50 µm pitch onto the hydrogel by a second solution discharge head filled with a cell-containing second solution, with which the hydrogels of the second layer were formed.

Then, as the repeat step, after impregnation with the first solution as above the same operation was repeated with the second solution discharge head while controlling the discharge position, so that hydrogels of the third layer and fourth layer were formed to complete finally a three-dimensional hydrogel constituted with 4 layers of hydrogels.

Lastly, the stack was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 6>

In this Example, a cell culture carrier in which a hydrogel containing cells was stacked on the support was produced using the production method for a cell culture carrier of the present invention. The basic procedure of this Example was the same as in Example 5. However, this Example was different from Example 5 in that the removal step was carried out after the gel formation step and the gel stack formation to remove an excessive reaction solution thereby reducing the thickness of the hydrogel layer. When the layer thickness of the stack is reduced and the pitch of the hydrogels is decreased, the distance between the cells in the hydrogels is shortened. As a result, the intercellular interaction is facilitated and the structure in the living body can be reproduced more accurately.

### (1) Gel formation step performing removal step, and solution stacking step and gel stack formation step comprising repeat step performing removal step

As the gel formation step, a hydrogel of 10 mm × 20 mm was formed by dropping droplets one by one at 100 µm pitch onto a support by a second solution discharge head filled with a cell-containing second solution.

After the formation, as the removal step, the hydrogel was gently immersed in a 35 mm dish filled with PBS(-) and washed. Then, as the solution stacking step, the hydrogel was placed in a dish containing the first solution and impregnated with the first solution.

Subsequently, as the gel stack formation step, 25 linear pattern hydrogels with a width of 200 µm and a length of 20 mm were formed at 400 µm intervals by dropping droplets one by one at 50 µm pitch onto the hydrogel by a second solution discharge head filled with a cell-containing second solution, with which the hydrogels of the second layer were formed.

As the repeat step, after performing the removal step in the same manner as above, and after impregnation with the first solution as above the same operation was repeated while controlling the discharge position with the second solution discharge head, so that hydrogels of the third layer and fourth layer were formed to complete finally a three-dimensional hydrogel constituted with 4 layers of hydrogels.

Lastly, after performing the removal step by gently immersing the stack in a 35 mm dish filled with PBS(-), the same was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 7>

In this Example, a cell culture carrier in which a hydrogel containing cells was stacked on the support was produced using the production method for a cell culture carrier of the present invention. The basic procedure of this Example was the same as in Example 4. However, this Example was different from Example 5 in that the plane formation step was carried out after the gel formation step and the gel stack formation.

### (1) Gel formation step performing plane formation step, and solution stacking step and gel stack formation step comprising repeat step performing plane formation step

A cell-containing second solution and a cell-free second solution were respectively placed into the liquid chambers of the second solution discharge heads. As the gel formation step, a hydrogel of 10 mm×20 mm was formed by dropping droplets one by one at 100 µm pitch onto a support by the second solution discharge head filled with the cell-containing second solution. Next, by the second solution discharge head filled with the cell-free second solution, droplets were dropped one by one onto the first layer hydrogel of 10 mm × 20 mm similarly at the pitch of 100 µm to improve the flatness of the first gel layer. Then, as the solution stacking step, the hydrogel was placed into the dish containing the first solution, and impregnated with the first solution.

Subsequently, as the gel stack formation step, 25 linear pattern hydrogels with a width of 200 µm and a length of 20 mm were formed at 400 µm intervals by dropping droplets one by one at 50 µm pitch onto the hydrogel by a second solution discharge head filled with a cell-containing second solution, with which the hydrogels of the second layer were formed.

As the repeat step, after performing the plane formation step in the same manner as above, and after impregnation with the first solution in the same manner as above, the same operation was repeated while tuning the discharge position of the second solution discharge head, to form hydrogels of the third layer, fourth layer, and subsequent layers, and finally to complete a three-dimensional hydrogel constituted with 10 layers of hydrogels.

Lastly, the stack was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 8>

In this Example, a cell culture carrier in which dot-shaped hydrogels containing cells were formed on the support was produced using the production method for a cell culture carrier of the present invention. The basic procedure of this Example was carried out the same as in Example 1. However, this Example was different from Example 1 in that the solution 1 and/or 2 did not contain cells, and the cell seeding step was carried out

### (1) Cell seeding step

To the cell culture carrier transferred to a 35 mm dish containing 2 mL of DMEM containing a 10 mass% FBS and a 1 mass% antibiotic, 20000 cells were seeded using a NHDF cell suspension prepared in the same manner as in Example 2 and placed in the incubator (described above) at 37°C in an environment of 5 volume% CO₂.

### <Example 9>

In this Example, a cell culture carrier in which a hydrogel containing cells was stacked on the support was produced using the production method for a cell culture carrier of the present invention. The basic procedure was the same as in Example 5. However, this Example was different from Example 4 in that each stacked hydrogel contains a different cell type, specifically that in the gel stack formation steps for the second and fourth layers the second solution containing human umbilical vein endothelial cells (hereinafter referred to as "HUVEC" or "HUVEC cells") instead of NHDF cells contained in the first layer and third layer was used.

### (1) Culture of HUVEC cells

HUVEC cells were cultured in the incubator (described above) at 37°C in an environment of 5 volume% CO₂ for 72 hours using an endothelial cell basic culture medium (Culture system containing EBM^{™}-2 Basal Medium (CC-3156), EGMTM-2 SingleQuots^{™} Supplements (CC-4176), produced by Lonza) in a 100-mm dish

### (2) Preparation of HUVEC cell-containing second solution

Part of the cell suspension obtained in the same manner as NHDF cells was transferred to an Eppendorf tube and centrifuged (device name: MiniSpin, manufactured by Eppendorf AG, 2.5 × 10³ rpm, 1 min), and then the supernatant was removed using a pipette. After the removal, the second solution for HUVEC cells was added to obtain a HUVEC cell-containing second solution which was composed of a cell suspension having a cell concentration of 1 × 10⁷ cells/mL and containing tetra-PEG- maleimidyl.

### (3) Hydrogel formation and gel lamination

An NHDF cell-containing second solution and an HUVEC cell-containing second solution were placed in the liquid chambers of the solution discharge heads shown in Figure 4. Hydrogels were formed by forming the first and third layers similar to Example 4 by the second solution discharge head 1 (0401) filled with the NHDF cell-containing second solution, and forming the second and fourth layers similar to Example 4 by the second solution discharge head 2 (0402) filled with the HUVEC cell-containing second solution. Finally, the hydrogels were transferred to a 35 mm dish containing 1 mL each of DMEM containing a 10 mass% FBS and a 1 mass% antibiotic, and an endothelial cell basic culture medium, and placed in the incubator (described above) at 37°C in an environment of 5 volume% CO₂.

### <Example 10>

In this Example, a cell culture carrier in which a hydrogel containing cells was stacked on the support was produced using the production method for a cell culture carrier of the present invention. The basic procedure was the same as in Example 5. However, this Example was different from Example 5 in that the discharge hole (0204) of the solution discharge head shown in Figure 2 was changed to a small hole so that the droplets to be dropped were made small.

### (1) Gel formation step, and solution stacking step and gel stack formation step comprising repeat step

As the gel formation step, a hydrogel of 10 mm × 20 mm was formed by dropping droplets one by one at 50 µm pitch onto a support by a second solution discharge head filled with a cell-containing second solution. Then, as the solution stacking step, the hydrogel was placed into the dish containing the first solution, and impregnated with the first solution.

Subsequently, as the gel stack formation step, 25 linear pattern hydrogels with a width of 200 µm and a length of 20 mm were formed at 400 µm intervals by dropping droplets one by one at 25 µm pitch onto the hydrogel by a second solution discharge head filled with a cell-containing second solution, with which the hydrogels of the second layer were formed.

Then, as the repeat step, after impregnation with the first solution as above the same operation was repeated while controlling the discharge position with the second solution discharge head, so that hydrogels of the third layer and fourth layer were formed to complete finally a three-dimensional hydrogel constituted with 4 layers of hydrogels.

Lastly, the stack was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 11>

In this Example, a cell culture carrier in which a hydrogel containing cells was stacked on the support was produced using the production method for a cell culture carrier of the present invention. The basic procedure was the same as in Example 5. However, this Example was different in that the fibrinogen is added as a cell acting additive to the first solution and the second solution.

### (1) Preparation of fibrinogen-containing first solution

The first solution containing 2% tetra-PEG-SH was prepared by dissolving 0.04 g of tetra-PEG-SH (described above) in 2 mL of PBS(-), and thereafter filtrating the solution with a filter with an average pore diameter of 0.2 µm (described above). Further, 0.02 g of fibrinogen (product name: Fibrinogen from bovine plasma, produced by Sigma-Aldrich) was added to the first solution, and dissolved with a microtube rotator (grade number: MTR-103, Airis 1 co.jp) to prepare the first solution containing 1% fibrinogen.

### (2) Preparation of thrombin-containing second solution for NHDF cells

The second solution containing 2% tetra-PEG- maleimidyl was prepared by dissolving 0.04 g of tetra-PEG- maleimidyl (described above) in 2 mL of PBS(-), and thereafter filtrating the solution with a filter with an average pore diameter of 0.2 µm (described above). Further, thrombin (product name: Thrombin from bovine plasma, produced by Sigma-Aldrich) was diluted with the above second solution to 20 U/mL to prepare a thrombin-containing second solution.

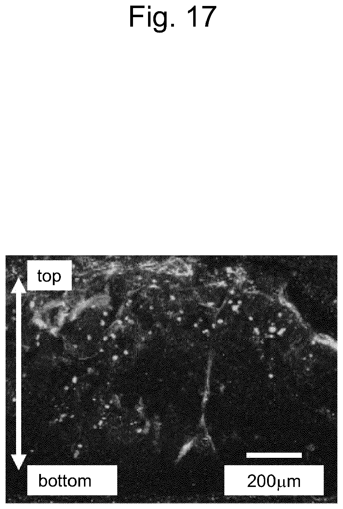

Figure 17 shows the state of fibrin in the hydrogel prepared in this Example. Fibrin is formed by a reaction of fibrinogen in the first solution and thrombin in the second solution. Although the target fibrin was formed in the hydrogel by a contact of the two solutions, the fibrin tended to be distributed slightly unevenly in the upper part.

### <Example 12>

In this Example, a cell culture carrier in which a hydrogel containing cells was stacked on the support was produced using the production method for a cell culture carrier of the present invention. The basic procedure was the same as in Example 11. However, different from Example 11, Matrigel (grade number: 354234, produced by Corning) was added as a cell acting additive to the first solution and the second solution to a concentration of 0.1 mass%.

### <Example 13>

In this Example, a cell culture carrier in which dot-shaped hydrogels containing cells were stacked on the support was produced using the production method for a cell culture carrier of the present invention. The basic procedure was the same as in Example 11. However, this Example was different in the shape of the formed hydrogels from Example 11 such that linear hydrogels were formed as the first layer, and dot-shaped hydrogels were stacked as the second layer.

### (1) Gel formation step, and solution stacking step and gel stack formation step

As the gel formation step, 20 linear hydrogels with a width of 200 µm and a length of 20 mm were formed at 400 µm intervals by dropping droplets one by one at 50 µm pitch onto a support by a second solution discharge head filled with a cell-containing second solution. Then, as the solution stacking step, the hydrogels were placed into the dish containing the first solution, and impregnated with the first solution.

Subsequently, as the gel stack formation step, a hydrogel in which 30 dot-shaped hydrogels were formed as the second layers of the respective linear dot-shaped hydrogels by dropping droplets one by one at 300 µm pitch onto the respective linear dot-shaped hydrogels in the hydrogel by a second solution discharge head filled with a cell-containing second solution.

Finally, the stack was transferred to a 35 mm dish containing 2 mL of DMEM containing a 10 mass% FBS and a 1 mass% antibiotic, and placed in the incubator (described above) at 37°C in an environment of 5 volume% CO₂.

### <Example 14>

In this Example, a cell culture carrier in which a membranous hydrogel containing cells was stacked on the support was produced using the production method for a cell culture carrier of the present invention. The basic procedure was the same as in Example 9. However, this Example was different from Example 9 in the shape of the hydrogel to be stacked, and all of the first layer to the fourth layer were stacked with membranous hydrogels.

### (1) Hydrogel formation and gel lamination

An NHDF cell-containing second solution and an HUVEC cell-containing second solution were placed in the liquid chambers of the solution discharge heads shown in Figure 4. Hydrogels in a size of 10 mm × 20 mm were formed in the first and third layers by dropping droplets one by one at 50 µm pitch onto the support and the hydrogel by the second solution discharge head 1 (0401) filled with the NHDF cell-containing second solution. Hydrogels in a size of 10 mm × 20 mm were formed in the second and fourth layers by dropping droplets one by one at 50 µm pitch onto the hydrogels by the second solution discharge head 2 (0402) filled with the HUVEC cell-containing second solution. Finally, the stack was transferred to a 35 mm dish containing 1 mL each of DMEM containing a 10 mass% FBS and a 1 mass% antibiotic, and an endothelial cell basic culture medium, and placed in the incubator (described above) at 37°C in an environment of 5 volume% CO₂.

In this example, a membranous hydrogel was formed from the first layer, and therefore whether or not a dot-shaped hydrogel was formed was not confirmed.

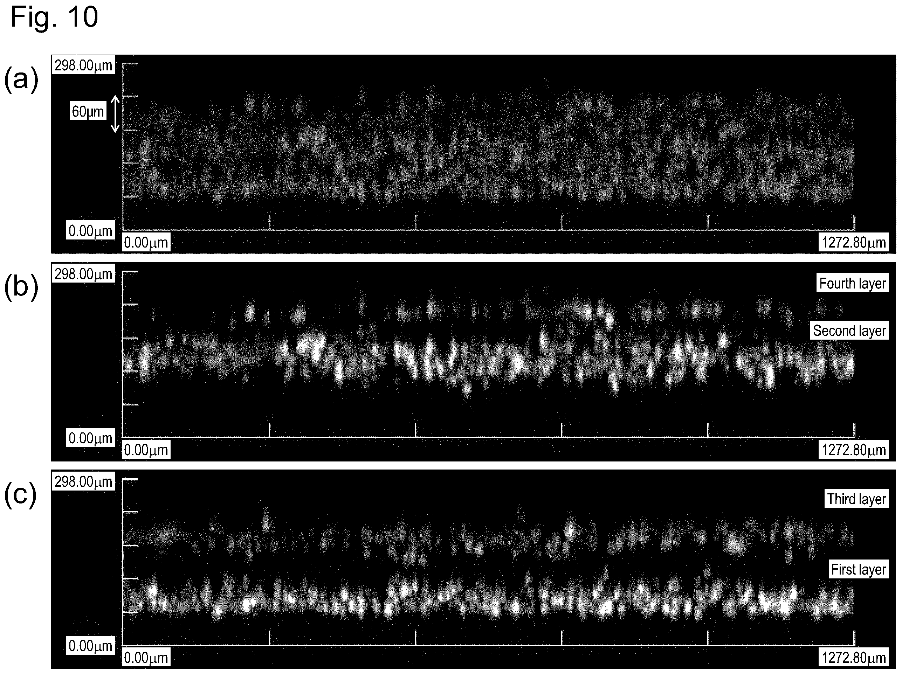

The NHDF cells were stained with a green fluorescent dye (trade name: Cell Tracker Green, produced by Thermo Fisher Scientific), and the HUVEC cells were stained with an orange fluorescent dye (trade name: Cell Tracker Orange, produced by Thermo Fisher Scientific), then the above operation was performed. One hour after the formation, the hydrogels were observed with a confocal microscope FV10 (described above). The observation results are presented in Figure 10.

### <Example 15>

In this Example, a cell culture carrier in which a hydrogel containing cells was stacked on the support was produced using the production method for a cell culture carrier of the present invention. The basic operation was the same as that described in Example 5. However, in this Example, a dispenser according to the gel extrusion method was used in place of the droplet formation device according to the inkjet method described in Example 5.

### <Example 16>

In this Example, a cell culture carrier in which a hydrogel containing cells was stacked on the support was produced using the production method for a cell culture carrier of the present invention. The basic procedure of this Example was in accordance with Example 7. However, this Example was different from Example 7 in that NIH/3T3 cells (Clone 5611, JCRB Cell Bank, hereinafter also referred to as "3T3") were used as the cells, the plane formation step was not performed, the number of layers was made as high as 10 or 20, and the culture medium (DMEM) was used instead of PBS to prepare the solution

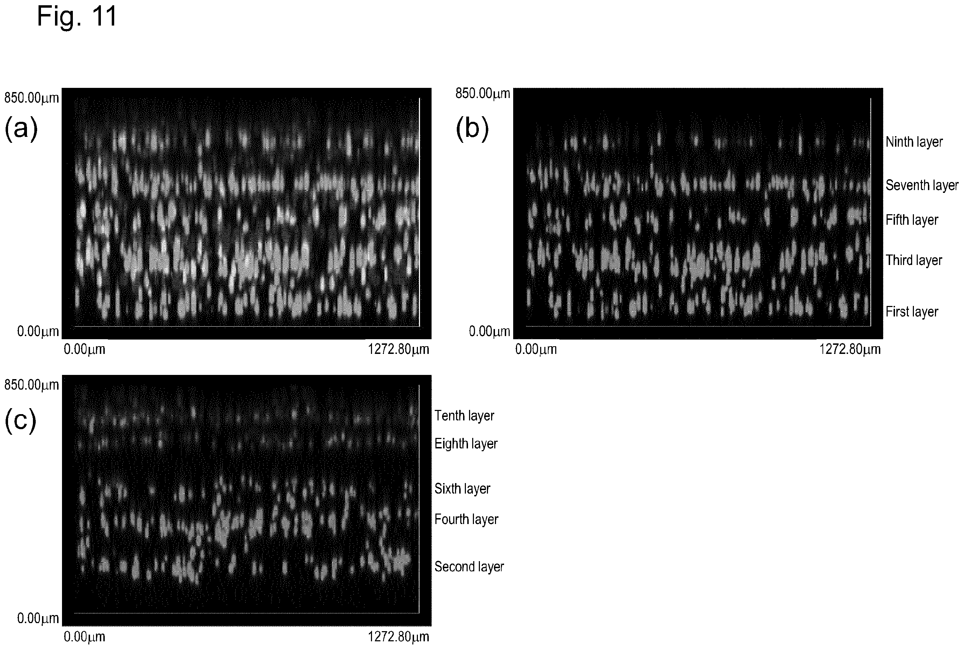

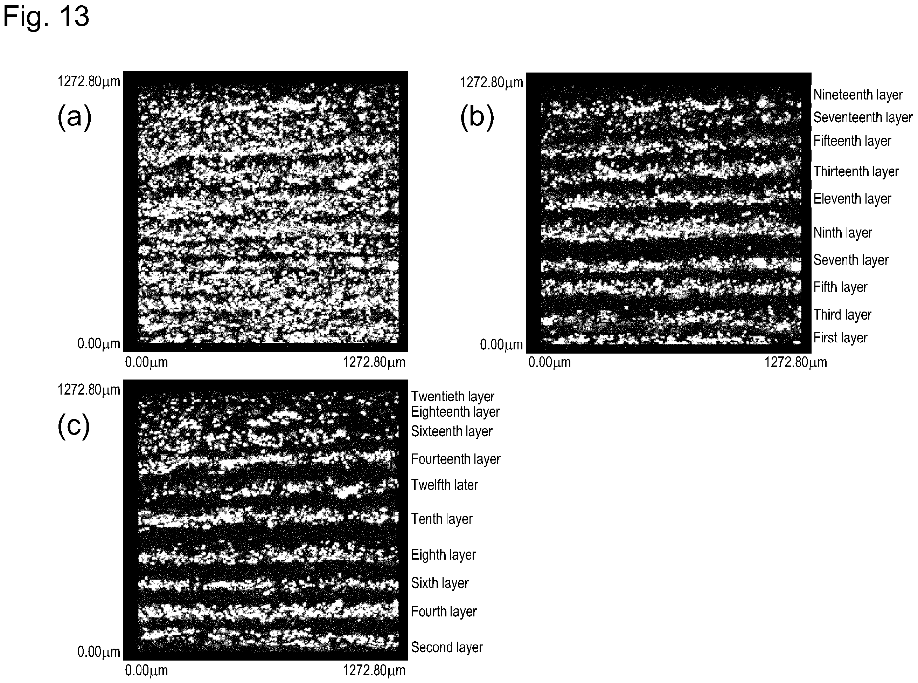

The cells in the odd-numbered layers were stained with a green fluorescent dye (trade name: Cell Tracker Green, produced by Thermo Fisher Scientific), and the cells in the even-numbered layers were stained with an orange fluorescent dye (trade name: Cell Tracker Orange, produced by Thermo Fisher Scientific). One hour after preparation, the cell culture carrier was observed with a confocal microscope FV10 (described above). The observation results of the 10 layer stacks are presented in Figure 11, and the observation results of the 20 layer laminate are presented in Figure 13.

### (1) Preparation of thrombin-containing first solution

The first solution containing 2% tetra-PEG-SH was prepared by dissolving 0.04 g of tetra-PEG-SH (described above) in 2 mL of DMEM, and thereafter filtrating the solution with a filter with an average pore diameter of 0.2 µm (described above). Further, thrombin (product name: Thrombin from bovine plasma, produced by Sigma-Aldrich) was diluted with the above second solution to 20 U/mL, thereby obtaining the thrombin-containing first solution.

### (2) Preparation of fibrinogen-containing second solution for 3T3 cells

A 1% fibrinogen solution was prepared by adding 0.02 g of fibrinogen (product name: Fibrinogen from bovine plasma, produced by Sigma-Aldrich) to 1 mL of DMEM, and dissolved with a microtube rotator (grade number: MTR-103, Airis1 co.jp). Then, tetra-PEG-maleimidyl (described above) was added, and the mixture was gently stirred and dissolved thereby obtaining a second solution containing 1% fibrinogen.

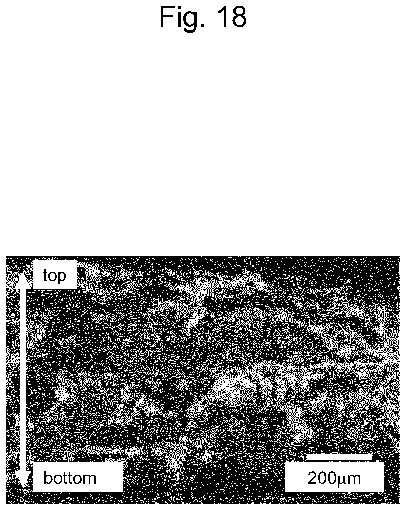

Figure 18 shows the state of fibrin in the hydrogel prepared in this Example. The target fibrin was formed by a contact of the two solutions in a hydrogel. When DMEM was used as the dispersion medium, fibrin was uniformly dispersed, and also a high cell survival rate was obtained.

### <Example 17>

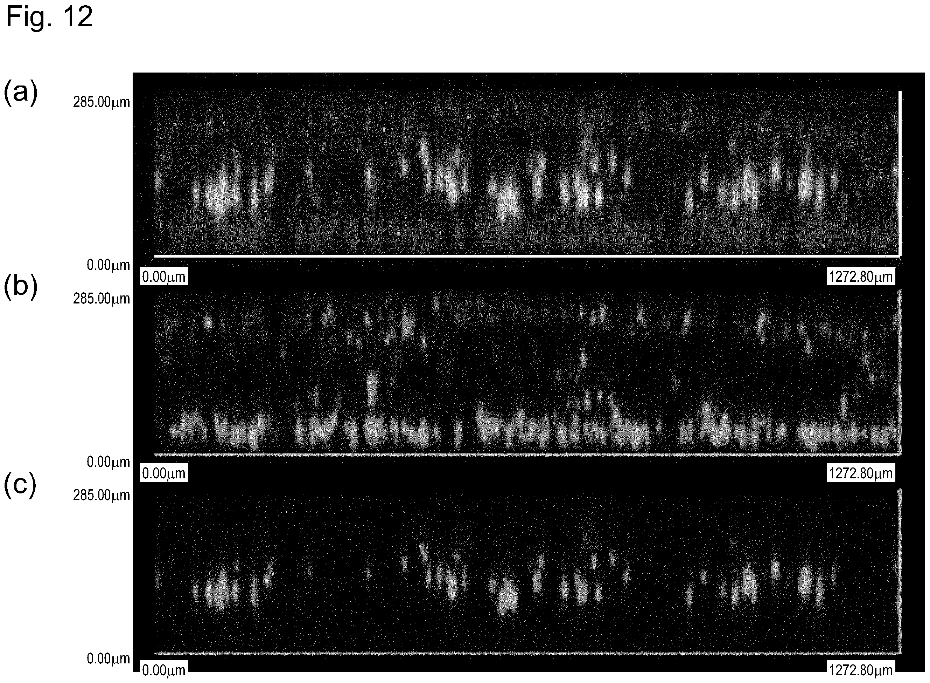

In this Example, a cell culture carrier in which a hydrogel containing cells was stacked on the support was produced using the production method for a cell culture carrier of the present invention. The preparation of the first solution and the second solution was in accordance with Example 16, and the basic procedure was in accordance with Example 9. However, in this Example, the structure of the cell culture carrier is different. Specifically, the first and third layers are membranous hydrogels containing NHDF cells, and the second layer is linear hydrogels. For the second layer, the cell acting gel formation step was performed to form a linear gel of fibrin gel containing HUVEC cells between linear hydrogels containing NHDF cells. One hour after preparation, the cell culture carrier was observed with a confocal microscope FV10 (described above). The observation results are presented in Figure 12.

### (1) Preparation of solution for HUVEC cell-containing cellular action gel

A 1% fibrinogen solution was prepared by adding 2 mL of PBS(-), and 0.02 g of fibrinogen to the first solution, and dissolving the mixture with a microtube rotator.

### (2) Production of solution for HUVEC cell-containing cellular action gel

Part of the cell suspension obtained in the same manner as NHDF cells was transferred to an Eppendorf tube, and centrifuged. Then the supernatant was removed using a pipette. After the removal, the 1% fibrinogen solution described above was added to yield a solution for HUVEC cell-containing cellular action gel consisting of a cell suspension with a cell concentration of 3 × 10⁶ cells/mL.

### (3) Hydrogel formation, cell acting gel formation, and gel lamination

The liquid chambers of the solution discharge heads were filled with an NHDF cell-containing second solution and an HUVEC cell-containing solution for cell acting gel respectively. The first layer similar to that in Example 4 was formed with the second solution discharge head filled with an NHDF cell-containing second solution. Then, as the solution stacking step, the hydrogel was put into a dish containing the first solution, and impregnated with the first solution.

Subsequently, as the gel stack formation step, a hydrogel construct of the second layer was formed by forming 25 linear pattern hydrogel constructs with a width of 200 µm and a length of 20 mm at 400 µm intervals by dropping droplets one by one at 90 µm pitch onto the hydrogel by the second solution discharge head filled with the cell-containing second solution. Further, the second layer was formed by forming a cell acting gel with a linear pattern of a width of 200 µm and a length of 20 mm by dropping droplets one by one at 90 µm pitch between the above linear-patterned hydrogel constructs of the second layer by the solution discharge head filled with the HUVEC cell-containing solution for cell acting gel. The third layer was formed in the same manner as the first layer, and finally the stack was transferred to a 35 mm dish containing 1 mL each of DMEM containing a 10 mass% FBS and a 1 mass% antibiotic, and an endothelial cell basic culture medium, and placed in the incubator (described above) at 37°C in an environment of 5 volume% CO₂.

### <Example 18>

In this Example, as in Example 16, a cell culture carrier in which 10 layers of hydrogels containing cells were stacked on the support was produced using the production method for a cell culture carrier of the present invention. The basic procedure of this Example was in accordance with Example 16. However, this Example was different from Example 16 in that HepG2 cells (JCRB Cell Bank, hereinafter also referred to as "HepG2") were used as the cells in place of 3T3 cells.

Even with this different kind of cell of HepG2, a high cell survival rate was obtained as in Example 16.

### <Comparative Example 1>

The basic procedure was carried out in the same manner as in Example 2. However, Comparative Example 1 was different from Example 2 in that the first solution and the second solution were prepared with the following materials instead of a multiple branching polymer comprising polyethylene glycol as the backbone, and an alginate gel was formed instead of tetra-PEG gel.

### (1) Preparation of first solution

After dissolving 0.584 g of calcium chloride (grade number: 192-13925, produced by Wako Pure Chemical Industries, Ltd.) (hereinafter referred to as "CaCl₂") in 100 mL of ultrapure water, the solution was filtrated with a filter with an average pore diameter of 0.2 µm (described above) to prepare a first solution consisting of a 100 mmol/L aqueous solution of CaCl₂.

### (2) Preparation of second solution

After dissolving 20 mg of sodium alginate (trade name: KIMICA ALGIN SKAT-ONE, produced by KIMICA Corporation) in 2 mL of ultrapure water, the solution was filtrated with a filter with an average pore diameter of 0.2 µm (described above) to prepare an aqueous solution of sodium alginate with a concentration of 1.0%.

### <Comparative Example 2>

The basic procedure was carried out in the same manner as in Example 2 and Comparative Example 1. However, Comparative Example 2 was different from Example 2 in that the first solution and the second solution were prepared with the following materials instead of a multiple branching polymer comprising polyethylene glycol as the backbone, and a fibrin gel was formed instead of tetra-PEG gel.

### (1) Preparation of first solution

A 1% fibrinogen-containing first solution was prepared by adding 0.02 g of fibrinogen (product name: Fibrinogen from bovine plasma, produced by Sigma-Aldrich) to 1.98 mL of PBS(-), and dissolved with a microtube rotator (grade number: MTR-103, Airis1 co.jp).

### (2) Preparation of second solution

A thrombin-containing second solution was prepared by diluting thrombin (product name: Thrombin from bovine plasma, produced by Sigma-Aldrich) with 2 mL of PBS(-) to 20 U/mL.

### <Reference Example 1>

The basic operation was in accordance with the method described in Example 5. In Reference Example 1, the procedure was manually performed instead of a droplet formation device based on the inkjet method.

### (Results)

### «Evaluation method»

The hydrogels formed by the methods of Examples and Comparative Examples were evaluated by the methods described below, and the results are summarized in Table 1-1 and Table 1-2 (herein, Table 1-1 and Table 1-2 are often collectively referred to as "Table 1").

**[Table 1-1]**

| | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Method | IJ | IJ | IJ | IJ | IJ | IJ | IJ | IJ | IJ | Small hole IJ | IJ |
| Gel | PEG | PEG | PEG | PEG | PEG | PEG | PEG | PEG | PEG | PEG | PEG |
| Number of cell species in gel | 0 | 1 | 1 | 1 | 1 | 1 | 1 | 1 | 2 | 1 | 1 |
| Cell species | - | NHDF | NHDF | NHDF | NHDF | NHDF | NHDF | NHDF | NHDF & HUVEC | NHDF | NHDF |
| Number of lamination layers | 1 | 1 | 2 | 2 | 4 | 4 | 10 | 1 | 4 | 4 | 4 |
| Gel shape | Dot | Dot | Dot | Line | Line | Line | Line | Dot | Line | Line | Line |
| Additional step | - | - | - | - | - | Removal | Plane formation | Cell seeding | - | - | - |
| Cell acting additive | - | - | - | - | - | - | - | - | - | - | Fibrin |
| Dispersion medium | PBS | PBS | PBS | PBS | PBS | PBS | PBS | PBS | PBS | PBS | PBS |
| Shape retention rate | Good | Good | Good | Good | Good | Good | Good | Good | Good | Good | Good |
| Dot | Good | Good | Good | Good | Good | Good | Good | Good | Good | Good | Good |
| Line | - | - | Good | Good | Good | Good | Good | - | Good | Good | Good |
| Borderless | - | - | Good | Good | Good | Good | Good | - | Good | Good | Good |
| Lamination | - | - | Good | Good | Good | Very good | Very good | - | Good | Very good | Good |
| Survival rate | - | Good | Good | Good | Good | Good | Good | Good | Good | Good | Good |
| Long-term survival rate | - | Good | Good | Good | Good | Good | Good | Good | Good | Good | Good |
| Morphology | - | - | - | - | - | - | - | - | - | - | Good |

**[Table 1-2]**

| | Example 12 | Example 13 | Example 14 | Example 15 | Example 16 | Example 17 | Example 18 | Comparative Example 1 | Comparative Example 2 | Reference Example 1 |
|---|---|---|---|---|---|---|---|---|---|---|
| Method | IJ | IJ | IJ | Dispenser | IJ | IJ | IJ | IJ | IJ | Manually |
| Gel | PEG | PEG | PEG | PEG | PEG | PEG | PEG | Alginate gel | Fibrin gel | PEG |
| Number of cell species in gel | 1 | 1 | 2 | 1 | 1 | 2 | 1 | 1 | 1 | 1 |
| Cell species | NHDF | NHDF | NHDF & HUVEC | NHDF | NIH/3T3 | NHDF & HUVEC | HepG2 | NHDF | NHDF | NHDF |
| Number of lamination layers | 4 | 2 | 4 | 4 | 10 or 20 | 3 | 10 | 1 | 1 | 4 |
| Gel shape | Line | Line/Dot | Membrane | Line | Line | Membrane/Line | Line | Dot | Dot | Line |
| Additional step | - | - | - | - | - | - | - | - | - | - |
| Cell acting additive | Matrigel | Fibrin | - | - | Fibrin | Fibrin | Fibrin | - | - | Fibrin |
| Dispersion medium | PBS | PBS | PBS | PBS | DMEM | DMEM | DMEM | Sodium alginate | PBS | PBS |
| Shape retention rate | Good | Good | Good | Good | Good | Good | Good | Poor | Good | Good |
| Dot-shaped | Good | Good | - | Poor | Good | Good | Good | Good | Poor | Poor |
| Linear | Good | Good | Good | Good | Good | Good | Good | - | - | Poor |
| Membranous | Good | - | Good | Good | Good | Good | Good | - | - | Good |
| Lamination | Good | Good | Good | Good | Very good | Good | Very good | - | - | Poor |
| Survival rate | Good | Good | Good | Good | Very good | Very good | Very good | Poor | Very good | Good |
| Long-term survival rate | Good | Good | Good | Good | Very good | Very good | Very good | Poor | Very good | Good |
| Morphology | Good | Good | - | - | Very good | Very good | Very good | - | - | Good |

In the table, IJ represents inkjet.

### (Shape retention)

In the hydrogel production process, when the final droplet of the reaction solution landed and the hydrogel was produced, the hydrogel was immediately transferred to a 35 mm dish, and DMEM containing 10 mass% FBS and a 1 mass% antibiotic was gently added thereto. The dish was placed in the incubator at 37°C in an environment of 5 volume% CO₂, and the diameter and thickness of the hydrogel were measured using a microscope (CKX41, manufactured by Olympus Corporation) immediately after the placement in the incubator and 3 days after the placement in the incubator. When the found diameter and thickness of the hydrogel were less than 75% of the values immediately after the placement in the incubator was rated as "poor", and when the same were not less than 75% it was rated as "good". In the case of a linear contiguous body with dot-shaped hydrogels (linear hydrogel), the evaluation was similarly performed based on the line width and thickness. In the case of a membranous contiguous body (membranous hydrogel), the evaluation was performed based on the thickness of the membrane. The results are presented in Table 1 as the "shape retention rate".

### (Planar placement: dot-shaped, linear, membranous)

In Examples and Comparative Examples, after preparation of the hydrogel, a culture medium was added in the same manner as in the above shape retention, the mixture was incubated at 37°C in an environment of 5 volume% CO₂, and 1 hour after the incubation the hydrogel was observed with a confocal microscope (FV10, manufacture by Olympus Corporation). It was checked whether or not dot-shaped hydrogels were placed at the specified pitch, or linear hydrogels or membranous (borderless) hydrogels were formed. The same observation method was used in the subsequent evaluation methods. The "pitch" mentioned above means the center-to-center distance between adjacent dot-shaped hydrogels.

As the evaluation criteria, when dot-shaped hydrogels were placed at a pitch of 400 µm, it was rated as Good, otherwise it was rated as Poor. The linear hydrogel was rated as Good, when it was confirmed that the dot-shaped hydrogels were contiguous, and rated as Poor, when it was not so confirmed. As for the membrane, when it was confirmed that dot-shaped hydrogels were contiguous in a 10 mm x 10 mm region in the X-Y plane, it was rated as Good, and when it was not so confirmed, it was rated as Poor. The results are presented in Table 1. In the table, "dot" represents dot-shaped hydrogel, "line" indicates linear hydrogel, and "borderless" indicates membranous hydrogel.

### (Stack)

In Examples and Comparative Examples, after preparation of the hydrogel, a culture medium was added in the same manner as in the above shape retention, the mixture was incubated at 37°C in an environment of 5 volume% CO₂, and after 1 hour the hydrogel was observed with a confocal microscope (FV10, manufacture by Olympus Corporation). It was checked whether or not dot-shaped hydrogels, linear hydrogels, or membranous hydrogels were formed meeting the thickness and the number of lamination layers designated in Table 2. The number of lamination layers in Table 2 means the number of layers formed.

As the evaluation criteria, when a layer was placed at the thickness (pitch) equal to or less than the value set forth in Table 2, or the number of lamination layers was equal to or more than the value set forth in Table 2, it was rated as Good, or Very good, otherwise it was rated as Poor. The results are presented in Table 1 as "Lamination".

**[Table 2]**

| Lamination | Thickness | Number of lamination layers |
|---|---|---|
| Good | 200pm | 2 |
| Very good | 50µm | 10 |

### (Survival rate)

### - Preparation of evaluation liquid for survival rate

An evaluation liquid for survival rate was prepared by adding 30 µL of PI (P1304MP, produced by Thermo Fisher Scientific) and 12 µL of Hoechst 33342 (H3570, produced by Thermo Fisher Scientific) to 60 mL of a culture medium corresponding to each sample,

### - Observation of cells

After the preparation of a hydrogel, a culture medium was added in the same manner as for the shape retention, and incubation was performed at 37°C in an environment of 5 volume% CO₂. Three days after the incubation 3 mL of the culture medium in a 3.5 cm-dish was replaced with the evaluation liquid for survival rate, and incubation was again performed for 1 hour in an incubator (described above) at 37°C in an environment of 5 volume% CO₂. The cells in the hydrogel were observed 1 hour after the incubation with a confocal microscope, and a three-dimensional image thereof was obtained.

### - Calculation of survival rate

Based on the three-dimensional image, the cells stained with PI were regarded as dead cells, and the cells stained with Hoechst 33342 were regarded as total cells, and the survival rate (%) was calculated by (total cell count - dead cell count) x 100/(total cell count). The results on 3T3 cells are presented in Table 3-1 and the results on HepG2 are presented in Table 3-2.

In a case where the cell survival rate was 60% or more 4 days after the incubation of the hydrogel, it was rated as Good; in a case where it was 80% or more, it was rated as Very good; and in a case where it was 60% or less, it was rated as Poor. The results are presented in Table 1 as "survival rate".

### (Long-term survival rate)

### - Observation of cells

After the preparation of a hydrogel, a culture medium was added in the same manner as for the shape retention above, and incubation was performed at 37°C in an environment of 5 volume% CO₂. After 7 days, 3 mL of the culture medium in the 3.5 cm-dish was replaced with 3 mL of the evaluation liquid for survival rate, and incubation was again performed for 1 hour in an incubator (described above) at 37°C in an environment of 5 volume% CO₂. The cells in the hydrogel were observed 1 hour after the incubation with a confocal microscope, and a three-dimensional image thereof was obtained.

### - Calculation of survival rate

Based on the three-dimensional image, the cells stained with PI were regarded as dead cells, and the cells stained with Hoechst 33342 were regarded as total cells, and the survival rate (%) was calculated by (total cell count - dead cell count) x 100/(total cell count). The results on 3T3 cells are presented in Table 3-1 and the results on HepG2 are presented in Table 3-2.

After preparing the hydrogel, the culture medium was added and cultured at 37°C in an environment of 5 volume% CO₂ for 7 days. In a case where the cell survival rate was 50% or more, it was rated as Good; in a case where it was 80% or more, it was rated as Very good; and in a case where it was 50% or less, it was rated as Poor. The results are presented in Table 1 as "long-term survival rate".

**[Table 3-1]**

| | NIH/3T3 survival rate | |
|---|---|---|
| | Day 4 | Day 7 |
| PBS | 64% | 51% |
| Culture medium | 96% | 93% |

**[Table 3-2]**

| | HepG2 survival rate | |
|---|---|---|
| | Day 3 | Day 7 |
| Culture medium | 87% | 82% |

### (Morphology)

In Examples and Comparative Examples with respect to the hydrogel to which a cell acting additive was added, after the final droplet of the reaction solution landed and a hydrogel was produced, a culture medium was added in the same manner as in the shape retention above, then the mixture was cultured at 37°C in an environment of 5 volume% CO₂. After 7 days, the morphology of the cells in the hydrogel was evaluated by whether the cells extended or not.

### - Preparation of solution for morphological observation

An evaluation liquid for morphological observation was prepared by adding 12 µL of Calcein AM (grade number: L3224, produced by Thermo Fisher Scientific) to 60 mL of a culture medium corresponding to each sample,

### - Observation of cells

After the evaluation of the above survival rate, 3 mL of the evaluation liquid for survival rate in the 3.5 cm dish was replaced with 3 mL of the above evaluation liquid for morphological observation and incubation was again performed for 1 hour in an incubator (described above) at 37°C in an environment of 5 volume% CO₂. The morphology of the cells in the hydrogel was observed with a confocal microscope 1 hour after the incubation.

From the cell morphology, if the cells extended, it was rated as Good, and if they did not extend, it was rated as Poor. Whether the cells extended or not was judged by whether or not pseudopodia of the cells were recognizable. The results are presented in Table 1 as "morphology".

### (Permeability)

The substance permeability of a hydrogel of a Preparation Example corresponding to each Example was evaluated by the method described below. The Examples corresponding to each Preparation Example as well as the results are summarized in Table 4.

**[Table 4]**

| | | Preparation Example 1 | Preparation Example 2 | Preparation Example 3 | Preparation Example 4 | Preparation Example 5 | Preparation Example 6 |
|---|---|---|---|---|---|---|---|
| Gel | | PEG | PEG | PEG | PEG | Alginate gel | Fibrin gel |
| Cell acting additive | | - | Fibrin | Matrigel | Fibrin | - | - |
| Dispersion medium | | PBS | PBS | PBS | DMEM | Sodium alginate | PBS |
| Corresponding Example | Example | 1-10,14,15 | 11,13, | 12 | 16-18 | - | - |
| | Reference Example | - | 1 | - | - | - | - |
| | Comparative Example | - | - | - | - | Comparison 1 | Comparison 2 |
| Permeability | | Good | Good | Good | Good | Poor | Good |

### - Preparation of evaluation gel for permeability

In this evaluation, the permeability of the hydrogel with a thickness of 1 mm placed in an insert (Falcon: 353097) corresponding to the 24-well plate (Falcon: 353047) was evaluated by a permeated volume of a fluorescent solution. For the staining solution, a stock solution prepared by dissolving 500 kDa of a fluorescent protein: Dextran Fluorescein Anionic (Invitrogen; D1823) in 2 mL of PBS(-) was diluted with one of various dispersion media to 1%, and used.

### - Evaluation of permeability

The above fluorescent solution was placed on the gel in the insert, and then a 24-well plate was filled with the same dispersion medium as the fluorescent solution, and left standing in an incubator at 37°C. Thereafter, the insert was taken out, and the solution in the 24-well plate was aliquoted in a 96-well plate for fluorescence measurement (Corning; 3694) at 50 µL for 4 wells. The fluorescence intensity thereof was measured with a plate reader (BioTek Instruments; Cytation 5). The measurement result was expressed by the amount of permeated substance in terms of molar concentration nM (nmol/m³). In a case where this value was 0.5 nM or more, the permeability was rated as Good; and in a case where the measurement was not possible, it was rated as Poor. The results are summarized in Table 4.

### (Time-dependent change of substance permeation amount)

In this evaluation, a 24-well plate and an insert corresponding to the 24-well plate were used. The pore size of the insert was 8 µm. The insert was set in the well of the 24-well plate to prepare a gel on the insert. For the gel, a PEG gel comprising fibrin as a cell acting additive using DMEM as a dispersion medium (which had the same quality as the gels used in Examples 16, 17, and 18, and did not contain cells) was used. The volume was determined from the bottom area of the insert and the gel thickness was adjusted in a range of from 1 to 4 mm. After preparation of the gel, 0.3 mL of a fluorescent solution was placed on the gel. For the fluorescent solution, a stock solution prepared by dissolving 500 kDa of Dextran Fluorescein Anionic in 2 mL of PBS(-) was diluted with DMEM to 1%, and used. After placing the fluorescent solution, 1 mL of a serum-free medium was placed in a 24-well plate. The inserts were taken out from the 24-well plate after standing in an incubator at 37°C for 2, 5, and 24 hours respectively using care not to spill the fluorescent solution on the gel, and the fluorescence intensity of the culture medium in the well was measured using a plate reader. The substance permeation amount was determined using the coefficient of the calibration curve obtained from the known amounts of the substance and the measured fluorescence intensities.

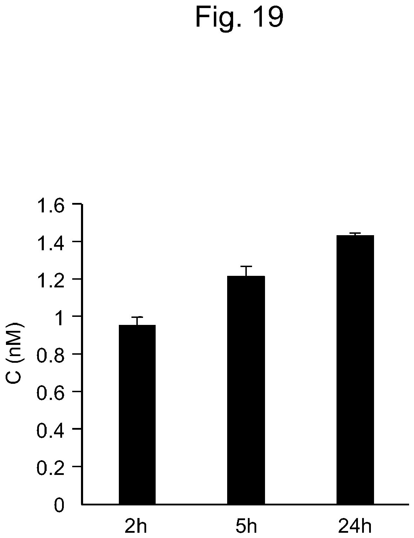

The results of the time dependence in protein permeation of the hydrogel were presented in Figure 19. After an elapse of 2 hours, the permeation amount was about 0.95 nM, which was much higher than 0.5 nM. The amount of substance permeated increased with the passage of time.

### «Evaluation»

It was made clear from the results of Examples 1 to 18, that the shape of the gel could be retained with respect to the tetra-PEG gel, while from the results of Comparative Example 1 with respect to the alginate gel that the shape retention of the gel was difficult due to detachment or collapse of the hydrogel.

Further, it was made clear from the results of Examples 1 to 14, that in the case of a tetra-PEG gel, formation of a dot-shaped hydrogel was possible, but in the case of a fibrin gel a dot-shaped hydrogel could not be formed due to slow gelation.

Figure 9 shows a perspective view of the fluorescently-stained image of the two-layer structure hydrogel in Example 3. Concerning the hydrogel shown in this figure, a membranous hydrogel was formed in the first layer, and thereon dot-shaped hydrogels were stacked as the second layer. From (a) it was known that dot-shaped hydrogels in the second layer shown in (b) were stacked on the membranous hydrogel in the first layer shown in (c). From this result, it was made clear that cell-containing dot-shaped hydrogels could be formed, to say the least, at a pitch of 400 µm.

Figure 10 shows a cross-sectional view of the fluorescently-stained image of the hydrogel prepared by stacking four layers in Example 14. It was confirmed from Figure 10 that cell-containing hydrogels could be stacked insofar as the thickness was at least 60 µm.

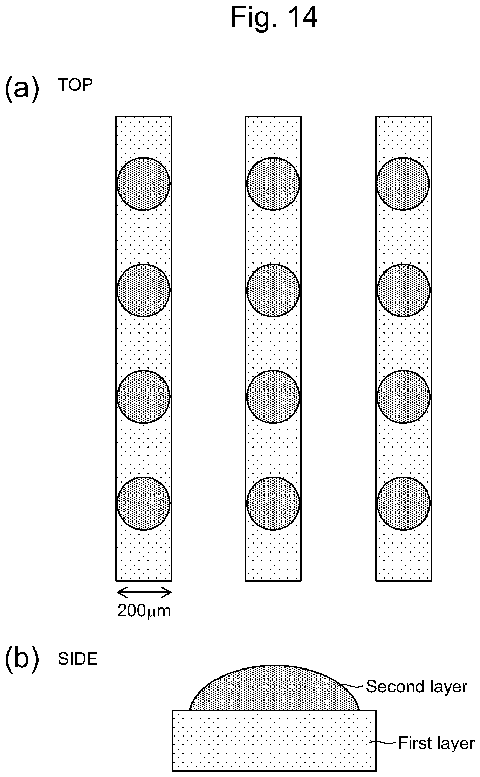

Figure 14 is a schematic view where tetra-PEG gels were formed and stacked in the first and second layers. When dot-shaped hydrogels that can maintain the shape can be formed as in the present invention, minute dot-shaped or fine linear hydrogels can be formed as shown in Figure 14. Therefore, hydrogels for cell culture can be formed and stacked in a desired shape with high resolution. In other words, it becomes possible to place cells three-dimensionally with high accuracy, and the structure in the living body can be reproduced more accurately.

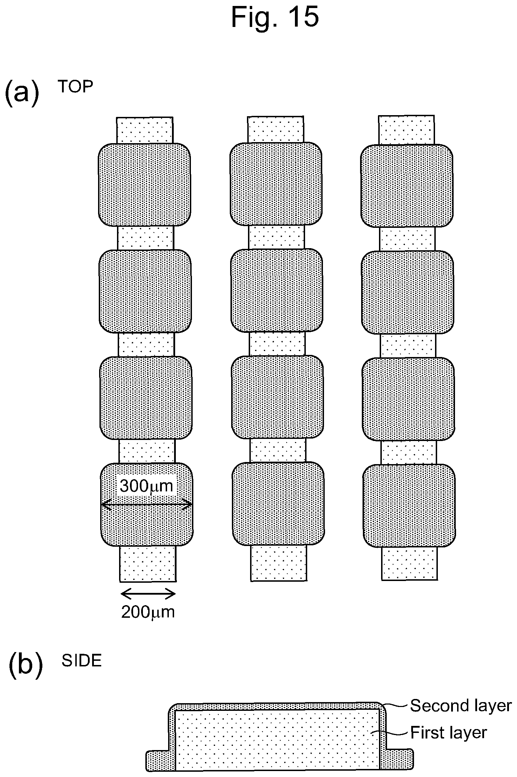

Meanwhile, Figure 15 is a schematic view for the case where a tetra-PEG gel is stacked in the first layer and an alginate gel or fibrin gel is stacked in the second layer. In the case of the alginate gel of Comparative Example 1, the shape of the gel cannot be maintained and the shape collapses. Further, in the case of the fibrin gel of Comparative Example 2, since the gelation time is long and the gel cannot be formed and stacked in an optional shape, it is not possible to form a high-resolution hydrogel.

It was confirmed from the results of Example 6 and Example 7 that the hydrogel could be stacked at a small pitch by carrying out the removal step or the plane formation step. Further, it was confirmed from Example 9, that lamination at a small pitch was possible even when a head with a small discharge hole was used. When lamination at a small pitch in the thickness direction is possible as in Examples 6, 7, and 9, hydrogels for cell culture can be stacked with high resolution, and the structure in the living body can be reproduced more accurately. Further, it was confirmed from Example 7, that when the plane formation step was carried out, 10 layers could be stacked, and the shape retaining effect of the tetra-PEG gel was high.

It was confirmed from Example 8, that the cells were viable not only when the cells were mixed in a second container, but also when the cells were seeded.

It was confirmed from Example 9 that a plurality of kinds of cells could be stacked.

It was confirmed from Examples 11, 12, and 13, that the cells extended by adding a cell acting additive.

From the results of Example 15 and Reference Example 1, it was difficult to shape dot-shaped hydrogels with a dispenser of a gel extrusion method, or by a manual technique, and unable to place dot-shaped hydrogels contiguously. Therefore, it was not possible to form an optional structure in which dot-shaped hydrogels are in contact with each other.

It was confirmed from the results of Example 16 and Example 18, that there was no disorder in each layer of gels placed three-dimensionally in the cell culture carrier in which 10 layers or 20 layers of cell-containing membranous hydrogels were stacked, and that its shape was retained. Further, it became clear that a cell acting additive present in the hydrogel was uniformly dispersed by using the dispersion medium of the first and/or second solution used for forming hydrogels as the medium for cell culture, and when the hydrogels contain cells, the survival rate of which also increased.

It became clear from the results of Examples 1 to 18, that the permeability of the gel was good in the tetra-PEG gel or the fibrin gel, while from the results of Comparatives Example 1 that the permeability of the gel was poor in the alginate gel, which was therefore disadvantageous as a cell culture carrier.

It became clear from the result of Figure 19 presenting the time-dependent change of the substance permeation amount, that the hydrogels of the present invention had a favorable substance permeability as a cell culture carrier, and did not inhibit delivery of nutrient components, etc. even when cells are cultured with a culture medium, etc.

### Advantageous Effects of Invention

According to the present invention, a cell culture carrier containing a hydrogel, wherein the cell culture carrier comprises a dot-shaped hydrogel with an optional diameter and thickness, and exhibits the shape retention rate after an elapse of 3 days from the formation of the hydrogel of 75% or more, can be obtained.

According to the present invention, a cell culture carrier which comprises a hydrogel containing a multiple branching polymer comprising polyethylene glycol as the backbone, and exhibits the shape retention rate after an elapse of 3 days from the formation of the hydrogel of 75% or more, can be obtained.

As obvious from the above results, it becomes possible according to the present invention to provide a production method for a cell culture carrier with a high shape retention rate, such a cell culture carrier, and a production device for a cell culture carrier, which have been unattainable by the conventional three-dimensional tissue model construction technology. In addition, since elements comprising cell survival rate, resolution, cell density, and cell species control can be established, a production method for a cell culture carrier that can place cell-containing gels three-dimensionally with high accuracy, allow cells to survive in the gels, and ensure high reproducibility, as well as a cell culture carrier, and production device for a cell culture carrier can be provided.

All publication, patents and patent applications cited herein shall be incorporated by citation directly herein.

## Claims

1. A production method for a cell culture carrier comprising:
a retention step of retaining a first solution containing a multiple branching polymer with one or more nucleophilic functional groups or electrophilic functional groups at a side chain(s) and/or a terminal(s), comprising polyethylene glycol as the backbone, and
a gel formation step of forming one or more hydrogels by landing a droplet of a second solution discharged from a droplet discharge device in the first solution so that the droplet contacts with the retained first solution,
wherein the second solution contains a multiple branching polymer with one or more other nucleophilic functional groups or electrophilic functional groups at a side chain(s) and/or a terminal(s), comprising polyethylene glycol as the backbone.

2. The production method for a cell culture carrier according to claim 1, wherein the first solution is retained on a support material.

3. The production method for a cell culture carrier according to claim 1 or 2, wherein the volume of the droplet is 9 pL or more and 900 pL or less.

4. The production method for a cell culture carrier according to any one of claims 1 to 3, wherein the volume of the hydrogel formed per one landing is 9 pL or more and 900 pL or less.

5. The production method for a cell culture carrier according to any one of claims 1 to 4, wherein all or part of the hydrogels are in contact with each other.

6. The production method for a cell culture carrier according to claim 5, wherein the hydrogels constitute a three-dimensional structure stacked so that all or part of two or more hydrogels overlap each other.

7. The production method for a cell culture carrier according to any one of claims 1 to 6 comprising a removal step of removing an unreacted first solution and second solution after the gel formation step.

8. The production method for a cell culture carrier according to any one of claims 1 to 7, comprising a plane formation step of improving the flatness of the hydrogel surface by stacking the first solution and the second solution.

9. The production method for a cell culture carrier according to any one of claims 1 to 8, comprising a cell seeding step of seeding cells on the hydrogels.

10. The production method for a cell culture carrier according to claim 9, wherein the cell seeding step is performed by the droplet discharge device.

11. The production method for a cell culture carrier according to any one of claims 1 to 10, comprising a cell acting gel formation step of stacking a cell acting gel containing a cell acting additive on the hydrogel.

12. The production method for a cell culture carrier according to claim 11, wherein the cell acting additive is an extracellular matrix protein and/or a growth factor.

13. The production method for a cell culture carrier according to any one of claims 1 to 12, wherein at least one or more of the first solution, the second solution, and the cell acting gel contains a dispersion medium.

14. The production method for a cell culture carrier according to claim 13, wherein the dispersion medium is a cell culture medium.

15. The production method for a cell culture carrier according to any one of claims 1 to 14, comprising:
a solution stacking step of stacking the first solution on the hydrogel or the cell acting gel, and
a gel stack formation step of landing the second solution by a droplet discharge device to contact with the stacked first solution to form a new stacked hydrogel.

16. The production method for a cell culture carrier according to claim 15, comprising a repeat step of repeating the solution stacking step and the gel stack formation step for a plurality of times.

17. The production method for a cell culture carrier according to any one of claims 1 to 16, wherein at least one or more of the first solution, the second solution, and the cell acting gel contains cells.

18. The production method for a cell culture carrier according to claim 17, comprising a cultivation step of culturing the cells.

19. The production method for a cell culture carrier according to claim 17 or 18, wherein the second solution contains at least two kinds of cells.

20. The production method for a cell culture carrier according to any one of claims 1 to 19, wherein the droplet discharge method of the droplet discharge device is an inkjet method.

21. A cell culture carrier consisting of one or more hydrogel consisting of a multiple branching polymer,
wherein the multiple branching polymer comprises polyethylene glycol with one or more nucleophilic functional groups and electrophilic functional groups at a side chain(s) and/or a terminal(s), as the backbone,
wherein the hydrogel has at least one structure selected from the group consisting of dot-shaped, linear, and membranous structures.

22. The cell culture carrier according to claim 21, comprising a support material that supports the hydrogel.

23. The cell culture carrier according to claim 21 or 22, wherein the shape retention rate of the hydrogel is 75% or more after an elapse of 3 days from the preparation of the hydrogel.

24. The cell culture carrier according to any one of claims 21 to 23, wherein an identical or two or more kinds of cells are contained in an identical hydrogel.

25. The cell culture carrier according to any one of claims 21 to 24, wherein the hydrogel has a three-dimensional structure in which all or part of two or more hydrogels are stacked to overlap each other.

26. The cell culture carrier according to any one of claims 21 to 25, wherein the volume of the hydrogel is 9 pL or more and 900 pL or less.

27. The cell culture carrier according to claim 26, wherein the hydrogel contains a cell acting additive.

28. The cell culture carrier according to claim 27, wherein the cell acting additive is an extracellular matrix protein and/or a growth factor.

29. The cell culture carrier according to any one of claims 21 to 28, wherein the hydrogel comprises a dispersion medium.

30. The cell culture carrier according to claim 29, wherein the dispersion medium is a cell culture medium.

31. A cell culture carrier production device at least comprising:
stage means of fixing a substrate,
retention means of retaining a first solution comprising a dispersion medium and a multiple branching polymer comprising as the backbone, either of polyethylene glycol or polyethylene glycol, with at least one electrophilic functional group at a side chain(s) and/or a terminal(s),
first solution discharge means of discharging the first solution to a substrate placed on the stage means,
retention means of retaining a second solution comprising dispersion medium and a multiple branching polymer comprising polyethylene glycol with the other functional group as the backbone,
second solution discharge means of discharging the second solution to a substrate placed on the stage means,
solution discharge control means of controlling the discharge of the respective solutions in the first solution discharge means and the second solution discharge means,
discharge position control means of calculating relative positions of the first solution discharge means, the second solution discharge means and the stage to control the discharge positions of the respective solutions, and
discharge order control means of controlling the discharge order of the first solution discharge means and the second solution discharge means.

32. The cell culture carrier production device according to claim 31, further comprising means of removing a multiple branching polymer comprising ungelled polyethylene glycol as the backbone, existing on the substrate.
